# EUROPÄISCHE PATENTSCHRIFT

(11) **EP 2 705 879 B1**
(45) Veröffentlichungstag und Bekanntmachung des Hinweises auf die Patenterteilung: **07.06.2017**
(21) Anmeldenummer: 13177837.5
(22) Anmeldetag: 24.07.2013
(51) Int. Cl.: A61K 8/37, A61K 8/39

(54) **ÖLKOMBINATION FÜR ANTITRANSPIRANT-ZUSAMMENSETZUNG MIT VERBESSERTER RÜCKSTANDSMASKIERUNG**
COMBINATION OF OILS FOR ANTIPERSPIRANT COMPOSITIONS WITH IMPROVED WHITE MARK PROTECTION
MÉLANGE D'HUILE POUR COMPOSITIONS ANTI-TRANSPIRANTES À MASQUAGE AMÉLIORÉ DES SELS RÉSIDUELS

(30) Priorität: 07.08.2012 DE 102012214015
(43) Veröffentlichungstag der Anmeldung: 12.03.2014
(73) Patentinhaber: Henkel AG & Co. KGaA, 40589 Düsseldorf (DE)
(72) Erfinder: Döring, Thomas, 41540 Dormagen (DE); Pfeifer, Stephanie, 53773 Hennef (DE)

(56) Entgegenhaltungen:
- EP-A1- 2 189 149
- WO-A1-97/34577
- US-A1- 2002 039 563
- US-A1- 2005 226 829
- US-A1- 2007 166 254
- U . KUX, M . HALLMANN, K . WARNKE, H . MIERTSCH, L . ENGFELDT, S . HOWE, M . MAURATH: "WASSERFREIE UND SILIKONÖLFREIE DEODORANTODER ANTITRANSPIRANTFORMULIERUNG IN STIFTFORM", COSMETICS: NEUES AUS DERMATOLOGIE, KOSMETIK UND WUNDVERSORGUNG, Bd. 3, Nr. 2/2004, 1, Februar 2004 (2004-02), Seiten 3-12, XP040423231, Deutschland

## Beschreibung

Die vorliegende Anmeldung betrifft wasserhaltige Antitranspirant-Zusammensetzungen, die eine verbesserte Rückstandsmaskierung aufweisen.

Handelsübliche schweißhemmende Zusammensetzungen, im Folgenden auch als Antitranspirantien bezeichnet, enthalten als Antitranspirant-Wirkstoff mindestens ein wasserlösliches adstringierendes anorganisches und organisches Salz des Aluminiums, Zirkoniums oder ausgewählte Aluminium-Zirkonium-Mischsalze. Die Antitranspirant-Wirkstoffe haben keinen direkten Einfluss auf die Tätigkeit der Schweißdrüsen, sondern minimieren durch Verengung der Ausflusskanäle die Schweißsekretion. Die Al-Salze bewirken dabei an den behandelten Hautflächen eine Schweißhemmung durch oberflächliche Verstopfung der Schweißdrüsenkanäle infolge von Al-Mucopolysaccharid-Niederschlägen. Antitranspirant-Zusammensetzungen werden üblicherweise im Bereich der Achselhöhlen appliziert und angewendet. Beim Antrocknen der Zusammensetzung auf der Haut oder auf der Kleidung, die mit der Haut nach Auftragen des Antitranspirants in Kontakt gekommen ist, bleibt das schweißhemmende Salz häufig als weißer Rückstand zu sehen. Dies tritt auch bei wasserhaltigen Zusammensetzungen auf, in denen das schweißhemmende Salz zunächst in gelöster Form vorliegt. Die weißen Rückstände werden vom Verbraucher als sehr negative Produkteigenschaft wahrgenommen. Zur Maskierung von Aluminiumsalz-Rückständen wasserhaltiger Zusammensetzungen sind im Stand der Technik sowohl wasserlösliche Bestandteile, wie insbesondere 1,2-Propylenglycol, als auch Öle, insbesondere Esteröle wie Isopropylpalmitat oder Alkylbenzoate, bekannt. Derartige Maskierungsmittel benetzen das schweißhemmende Salz und verdunsten auch nach dem Auftragen auf die Haut nicht, wie es zum Beispiel Wasser und Cyclomethicone tun. Dadurch trocknet das schweißhemmende Salz deutlich langsamer, und das Auftreten sichtbarer Rückstände wird verzögert. Die Maskierung kann weiter verbessert werden, indem ein Maskierungsmittel ausgewählt wird, dessen Brechungsindex no im Bereich des Brechungsindex n_{D} typischer schweißhemmender Salze bzw. typischerweise eingesetzter wässriger Lösungen von schweißhemmenden Salzen liegt, also im Bereich von n_{D}²⁰ = 1,4 bis 1,5. Öle mit hoher maskierender Wirkung haben den Nachteil, dass sie zu einer Anschmutzung der Kleidung ("fabric staining") führen können.

Antitranspirant-Zusammensetzungen sind in diversen Darreichungsformen erhältlich, zum Beispiel als mit Treibgas versprühbare Zusammensetzung. Derartige Zusammensetzungen werden meist in Spraydosen aus Aluminium oder (seltener) Weißblech abgefüllt, die durch eine Innenlackierung vor Korrosion geschützt sind. Trotz einer solchen Schutzlackierung kann es aber immer zu Korrosionsschäden kommen. Ein weiteres Problem derartiger Produkte besteht darin, dass das Ventil verstopft. Eine korrosionshemmende und/oder die Ventilverstopfung reduzierende Zusammensetzung wäre daher für diese speziellen Darreichungsformen wünschenswert. Im Stand der Technik ist das Problem der Formulierung von möglichst rückstandsfreien Antitranspirantsprodukten bekannt (US2007-0166254, EP2189149, US20020039563, WO9734577). Es besteht daher ein ständiger Bedarf an Antitranspirant-Zusammensetzungen mit hoher Rückstandsmaskierung, reduzierten sichtbaren Rückständen, reduzierter Klebrigkeit, reduzierter Faseranschmutzung, verbesserter Temperaturstabilität, verlängerter Parfümhaftung, und - bei versprühbaren Zusammensetzungen - reduzierter Korrosionswirkung und reduzierter Ventilverstopfung.

Eine Aufgabe der vorliegenden Erfindung war es, Antitranspirant-Zusammensetzungen mit hoher Rückstandsmaskierung und reduzierten sichtbaren Rückständen bereitzustellen. Eine weitere Aufgabe der vorliegenden Erfindung war es, Antitranspirant-Zusammensetzungen mit reduzierter Klebrigkeit und reduzierter Faseranschmutzung bereitzustellen.

Eine weitere Aufgabe der vorliegenden Erfindung war es, wasserhaltige Antitranspirant-Zusammensetzungen mit verbesserter Temperaturstabilität, insbesondere bei niedrigen Temperaturen, verlängerter Parfümhaftung und reduzierter Korrosionswirkung bereitzustellen.

Überraschend wurde gefunden, dass die gestellten Aufgaben durch eine bestimmte Kombination von Ölkomponenten in einer definierten Matrix gelöst werden.

In den Beiträgen "Wasserfreie und silikonölfreie Deodorant- oder Antitranspirantformulierungen in Stiftform" und "Wasserfreie und silikonölfreie Deodorant- oder Antitranspirantformulierungen in Aerosolform" der Publikation "Neues aus Dermatologie, Kosmetik und Wundversorgung", Nr. 3, Februar 2004, herausgegeben von der Beiersdorf AG, Hamburg, sind schweißhemmende Zusammensetzungen, enthaltend PPG-14 Butylether und C12-15-Alkylbenzoat, offenbart. Auch US 2007/0166254A1, EP 2189149A1 und US 2002/0039563A1 offenbaren schweißhemmende Zusammensetzungen, enthaltend PPG-14 Butylether und C12-15-Alkylbenzoat WO 97/34577A1 offenbart schweißhemmende Zusammensetzungen in Stiftform, die zur Rückstandsmaskierung ein nicht-flüchtiges Öl mit einem Brechungsindex von mindestens 1,4460, darunter PPG-14 Butylether und verschiedene Alkylbenzoate, enthalten. US 2005/0226829A1 offenbart einen Antitranspirantstift, der zur Rückstandsmaskierung Prpylenglycoldibenzoat enthält.

Ein Gegenstand der vorliegenden Anmeldung ist daher eine schweißhemmende Zusammensetzung zur persönlichen Körperpflege, konfektioniert als Nonaerosol, Stift, Soft Solid, Creme, Gel, nicht-versprühbare oder versprühbare Suspension, nicht-versprühbare Lösung oder auf einem Substrat imprägniert, enthaltend - jeweils bezogen auf ihr Gewicht ggf. nach Abzug flüchtiger Treibmittel -
a) mindestens einen schweißhemmenden Wirkstoff,
b) 0 - 7 Gew.-%, bevorzugt 0 - 3 Gew.-%, freies Wasser,
c) eine Mischung mindestens zweier unter Normalbedingungen flüssiger Öle als Träger,
wobei die Zusammensetzung
c1) 5 bis 30 Gew.-% PPG-14 Butylether und
c2) 0,5 bis 20 Gew.-% mindestens eines Öls aus der Gruppe der Alkylbenzoate, der Alkylendibenzoate, der alkoxylierten Alkylbenzoate, der Polyalkylenoxid-Dibenzoate oder deren Mischungen enthält, mit der Maßgabe, dass der Gewichts-Anteil von Ölkomponenten mit einem Brechungs- index ≥ 1,465 weniger als die Hälfte des Gesamtgewichts aller Ölkomponenten c) ausmacht,
und weiterhin mit der Maßgabe, dass die Zusammensetzung 0,75 bis 15 Gew.-%, Propylenglycoldibenzoat enthält.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Alle Mengenangaben beziehen sich, soweit nicht anders angegeben, auf das Gesamtgewicht der erfindungsgemäßen Antitranspirant-Zusammensetzung. Eventuell zugesetzte Treibmittel zählen nicht zur erfindungsgemäßen Antitranspirant-Zusammensetzung, daher beziehen sich alle Mengenangaben auf das Gesamtgewicht der treibmittelfreien Antitranspirant-Zusammensetzung, sofern nichts anderes angegeben ist.

"Freies Wasser" im Sinne der vorliegenden Anmeldung ist Wasser, das nicht in Form von Kristallwasser, Hydratationswasser oder ähnlich molekular gebundenem Wasser in der Antitranspirant-Zusammensetzung enthalten ist. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar. Freies Wasser ist beispielsweise solches Wasser, das als Lösemittel, als Gelaktivator oder als Lösemittelbestandteil anderer Wirkstoffe zur erfindungsgemäßen Zusammensetzung zugegeben wird.

Die erfindungsgemäßen Antitranspirant-Zusammensetzungen enthalten, bezogen auf ihr Gesamtgewicht, 0 bis 7 Gew.-% freies Wasser. Erfindungsgemäß bevorzugte Antitranspirant-Zusammensetzungen enthalten, bezogen auf ihr Gesamtgewicht, 0 bis 6 Gew.-% freies Wasser, bevorzugt 0 - 5 Gew.-%, besonders bevorzugt 0 - 4 Gew.-%, außerordentlich bevorzugt 0 - 3 Gew.-% freies Wasser.

Die erfindungsgemäßen Zusammensetzungen enthalten mindestens einen schweißhemmenden Wirkstoff. Bevorzugte Antitranspirant-Wirkstoffe sind ausgewählt aus den wasserlöslichen adstringierenden anorganischen und organischen Salzen des Aluminiums, Zirkoniums und Zinks bzw. beliebigen Mischungen dieser Salze.

Erfindungsgemäß wird unter Wasserlöslichkeit eine Löslichkeit von wenigstens 3 Gew.-% bei 20 °C verstanden, das heißt, dass Mengen von wenigstens 3 g des Antitranspirant-Wirkstoffs in 97 g Wasser bei 20 °C löslich sind. Erfindungsgemäß bevorzugt wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), dehydratisierter Alaun (KAl(SO₄)₂ mit null bis 11 Mol Kristallwasser), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat, Zirconyloxyhalogeniden, insbesondere Zirconyloxychloriden, Zirconylhydroxyhalogeniden, insbesondere Zirconylhydroxychloriden (Zirkoniumchlorohydrat).

Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Aktivierte Aluminium- und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminium- und Aluminium-Zirconiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminium- oder Aluminium-Zirconiumsalzes, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 -42 Gew.-%, aktiviertes schweißhemmendes Aluminium- oder Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole.

Weitere bevorzugte schweißhemmende Wirkstoffe sind basische Calcium-Aluminiumsalze. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, die mit Salzen von Aminosäuren, insbesondere mit Alkali- und Erdalkaliglycinaten, gepuffert sind. Weitere bevorzugte schweißhemmende Wirkstoffe sind aktivierte Aluminium- oder Aluminium-Zirconiumsalze, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(Al+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48-78 Gew.-% (USP), bevorzugt 66-75 Gew.-% eines aktivierten Aluminium-oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminium- oder Aluminium-Zirconiumsalze enthalten 5 - 78 Gew.-% (USP) eines aktivierten schweißhemmenden Aluminium-oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Strontiumsalz in einer solchen Menge, um ein Sr:(Al+Zr)-Gewichtsverhältnis von 1:1 - 1:28 und bevorzugt 1:2 - 1:25 bereitzustellen. Besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 -16 Gew.-%, bevorzugt 4 -13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt. Erfindungsgemäß besonders bevorzugte Zirconium-freie Aluminiumsesquichlorohydrate weisen ein molares Metall-zu-Chlorid-Verhältnis von 1,5:1-1,8:1 auf. Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein Al:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein Al:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen.

Erfindungsgemäß bevorzugte Zirconiumsalze haben die allgemeine Formel ZrO(OH)₂₋ₐClₐ · x H₂O mit a = 1.5 - 1.87; x = 1 - 7, wobei a und x rationale Zahlen sind.

Bevorzugte Aluminiumzirconiumsalze weisen ein molares Metall-zu-Chlorid-Verhältnis von 0,9 - 1,3, bevorzugt 0,9 - 1,1, besonders bevorzugt 0,9 - 1,0, auf.

Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:Cl = 1,1 - 0,9), und besonders bevorzugt m = 1,00 - 1,11 (entsprechend M:Cl = 1,0 - 0,9), weiterhin sehr bevorzugt m = 1,02 - 1,11 (entsprechend M:Cl = 0,98 - 0,9) sowie sehr bevorzugt m = 1,04 - 1,11 (entsprechend M:Cl = 0,96 - 0,9).

Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrat (Al:Zr = 2-6; M:Cl = 0.9-1.3), insbesondere Salze mit einem molaren Metall-zu-Chlorid-Verhältnis von 0,9 - 1,1, bevorzugt 0,9 -1,0.

Weiterhin erfindungsgemäß bevorzugt sind Aluminiumzirconiumchlorohydrat-Glycin-Salze, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5) : 1 und ein molares Verhältnis von Betain zu Glycin von mindestens 0,001 : 1 auf. In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet).

Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt.

Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind.

Besonders bevorzugte erfindungsgemäße Zusammensetzungen sind dadurch gekennzeichnet, dass der mindestens eine Antitranspirant-Wirkstoff in einer Menge von 5 - 40 Gew.-%, bevorzugt 10 - 35 Gew.-%, besonders bevorzugt 15 - 28 Gew.-% und außerordentlich bevorzugt 23-27 Gew.-%, enthalten ist, bezogen auf das Gesamtgewicht der kristallwasserfreien Aktivsubstanz (USP) in der Zusammensetzung.

Da die Rückstands-maskierende Wirkung der erfindungsgemäßen Ölkombination aus Öl, PPG-14 Butylether und ggf. alkoxylierten Alkyl(en)(di)benzoaten besonders vorteilhaft bei Aluminiumzirconiumsalzen einzusetzen ist, enthalten bevorzugte erfindungsgemäße Zusammensetzungen-jeweils bezogen auf ihr Gewicht ggf. nach Abzug flüchtiger Treibmittel -
a) mindestens ein Aluminiumzirconiumsalz als schweißhemmenden Wirkstoff,
b) 0 - 7 Gew.-%, bevorzugt 0 - 3 Gew.-%, freies Wasser,
c) eine Mischung mindestens zweier unter Normalbedingungen flüssiger Öle als Träger,
wobei die Zusammensetzung
c1) 5 bis 30 Gew.-% PPG-14 Butylether und
c2) 0,5 bis 20 Gew.-% mindestens eines Öls aus der Gruppe der Alkylbenzoate, der Alkylendibenzoate, der alkoxylierten Alkylbenzoate, der Polyalkylenoxid-Dibenzoate oder deren Mischungen enthält, mit der Maßgabe, dass der Gewichts-Anteil von Ölkomponenten mit einem Brechungs- index ≥ 1,465 weniger als die Hälfte des Gesamtgewichts aller Ölkomponenten c) ausmacht,
und weiterhin mit der Maßgabe, dass die Zusammensetzung 0,75 bis 15 Gew -%, Propylenglycoldibenzoat enthält.

Die erfindungsgemäßen Zusammensetzungen können in einer weiteren besonders bevorzugten Ausführungsform sowohl mindestens einen Deodorant- als auch mindestens einen Antitranspirant-Wirkstoff enthalten.

Als weitere Inhaltstoffe enthalten die erfindungsgemäßen Zusammensetzungen eine Mischung mindestens zweier unter Normalbedingungen flüssiger Öle als Träger.

Die erfindungsgemäßen Zusammensetzungen enthalten mindestens ein Öl als Trägerfluid.

Erfindungsgemäß bevorzugte Antitranspirant-Zusammensetzungen enthalten insgesamt 30 - 95 Gew.-%, bevorzugt insgesamt 40 - 93 Gew.-%, besonders bevorzugt insgesamt 50 - 90 Gew.-%, außerordentlich bevorzugt insgesamt 55 - 85 Gew.-%, jeweils bezogen auf die gesamte Zusammensetzung, an mindestens einem unter Normalbedingungen flüssigen kosmetischen Öl. Weitere erfindungsgemäß bevorzugte Antitranspirant-Zusammensetzungen enthalten insgesamt 35 - 60 Gew.-%, bevorzugt 40 - 55 Gew.-%, besonders bevorzugt 45 - 50 Gew.-%, jeweils bezogen auf die gesamte Zusammensetzung, an mindestens einem unter Normalbedingungen flüssigen kosmetischen Öl. Auch eine Gesamtmenge an unter Normalbedingungen flüssigen kosmetischen Ölen von 42, 43, 44, 46, 47, 48, 49, 51, 52, 53, 55, 58, 60, 63, 65, 68, 70, 73, 75, 78 oder 80 Gew.-%, jeweils bezogen auf die gesamte Zusammensetzung, kann erfindungsgemäß besonders bevorzugt sein, wobei eine Gesamtmenge von 45 - 55 Gew.-% besonders bevorzugt ist.

Die erfindungsgemäßen Zusammensetzungen können nur zwei (s.u.), aber auch drei, vier, fünf, sechs oder noch mehr verschiedene unter Normalbedingungen flüssiger Öle als Träger enthalten. Erfindungsgemäß enthalten die Zusammensetzungen - bezogen auf ihr Gewicht - 5 bis 30 Gew.-% PPG-14 Butylether (c1) und 0,5 bis 20 Gew.-% mindestens eines Öls aus der Gruppe der Alkylbenzoate, der Alkylendibenzoate, der alkoxylierten Alkylbenzoate, der Polyalkylenoxid-Dibenzoate oder deren Mischungen (c2), wobei 0,75 bis 15 Gew.-%, Propylenglycoldibenzoat enthalten sind. Dabei werden die Ölkomponenten so ausgewählt, dass der Anteil von Ölkomponenten c) mit einem Brechungsindex ≥ 1,465 weniger als die Hälfte (< 50 Gew.-%) der Gesamtmenge aller Ölkomponenten ausmacht. Der PPG-14 Butylether (c1) hat einen Brechungsindex < 1,465, so dass bei erfindungsgemäßen Zusammensetzungen, die nur die beiden Öle c1 und c2 enthalten, Art und Menge der Ölkomponenten c2 so gewählt werden, dass die vorstehende Bedingung erfüllt ist. Bei erfindungsgemäßen Zusammensetzungen, die drei oder mehr Öle enthalten, kann durch entsprechende Wahl der weiteren Öle die vorstehende Bedingung eingehalten werden. Erfindungsgemäß bevorzugte Zusammensetzungen enthalten das Öl c1 innerhalb engerer Mengenbereiche. Hier sind erfindungsgemäß bevorzugte Zusammensetzungen dadurch gekennzeichnet, dass sie 7,5 bis 27,5 Gew.-%, vorzugsweise 10 bis 25 Gew.-%, weiter bevorzugt 12,5 bis 22,5 Gew.-%, noch weiter bevorzugt 15 bis 20 Gew.-% und insbesondere 16 bis 17,5 Gew.-% PPG-14 Butylether enthalten.

Auch die Ölkomponente(n) c2 ist/sind vorzugsweise innerhalb engerer Mengenbereiche in den erfindungsgemäßen Zusammensetzungen enthalten. Hier sind erfindungsgemäß bevorzugte Zusammensetzungen dadurch gekennzeichnet, dass sie 0,75 bis 15 Gew.-%, vorzugsweise 1,0 bis 10 Gew.-%, weiter bevorzugt 1,25 bis 7,5 Gew.-%, noch weiter bevorzugt 1,5 bis 5 Gew.-% und insbesondere 1,75 bis 3,5 Gew.-% mindestens eines Öls aus der Gruppe der Alkylbenzoate, der Alkylendibenzoate, der alkoxylierten Alkylbenzoate, der Polyalkylenoxid-Dibenzoate oder deren Mischungen enthalten.

Erfindungsgemäß bevorzugte Öle c2 sind ausgewählt aus den Benzoesäureestern von linearen oder verzweigten C₈₋₂₂-Alkanolen. Bevorzugte Alkylbenzoate sind Dodecylbenzoat, Terdecylbenzoat, Tetradecylbenzoat, Pentadecylbenzoat, Hexadecylbenzoat, Octdecylbenzoat, 2-Methyl-heptadecylbenzoat, Octyldodecylbenzoat. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD, wobei Benzoesäure-C12-C15-alkylester außerordentlich bevorzugt sind.

Besonders bevorzugte erfindungsgemäße Zusammensetzungen sind dadurch gekennzeichnet, dass sie 0,75 bis 15 Gew.-%, vorzugsweise 1,0 bis 10 Gew.-%, weiter bevorzugt 1,25 bis 7,5 Gew.-%, noch weiter bevorzugt 1,5 bis 5 Gew.-% und insbesondere 1,75 bis 3,5 Gew.-% Octyldodecylbenzoat, Isostearylbenzoat, Dodecylbenzoat, Tridecylbenzoat, Tetradecylbenzoat, Pentadecylbenzoat, Octyldodecenyldibenzoat, Isostearenyldibenzoat, Dodecenyldibenzoat, Tridecenyldibenzoat, Tetradecenyldibenzoat, Pentadecenyldibenzoat, Ethylenglycoldibenzoat, Butylenglycoldibenzoat oder deren Mischungen enthalten.

Bei den alkoxylierten Alkylbenzoaten ist die Alkylgruppe mit einer Alkoxygruppe terminiert, die vorzugsweise ausgerwählt ist aus Ethoxy- und Propoxygruppen. Vorzugsweise weisen entsprechende Verbindungen 1 bis 30 AO-Einheiten auf, wobei Verbindungen mit 2 bis 25, vorzugsweise 3 bis 20, weiter bevorzugt 4 bis 15 und insbesondere 5 bis 10 AO-Gruppierungen bevorzugt sind. Ganz besonders bevorzugte Verbindungen enthalten 2 bis 25, vorzugsweise 3 bis 20, weiter bevorzugt 4 bis 15 und insbesondere 5 bis 10 EO-Gruppierungen.

Weiterhin können (Poly)alkylenglycole mit beidseitig endständiger Benzoatgruppierung (Polyalkylenoxid-Dibenzoate) eingesetzt werden. Diese lassen sich durch die allgemeine Formel (I) beschreiben 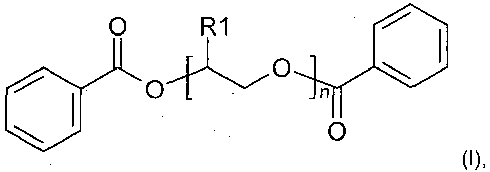 in der R1 für -H oder -CH₃ und n für ganze Zahlen von 1 bis 100, vorzugsweise für 1 bis 10, besonders bevorzugt für 1 bis 4 steht. Besonders bevorzugte Vertreter der Formel (I) sind
- Ethylenglycoldibenzoat (R1 = -H, n = 1)
- Diethylenglycoldibenzoat (R1 = -H, n = 2)
- Triethylenglycoldibenzoat (R1 = -H, n = 3)
- Tetraethylenglycoldibenzoat (R1 = -H, n = 4)
- Pentaethylenglycoldibenzoat (R1 = -H, n = 5)
- Hexaethylenglycoldibenzoat (R1 = -H, n = 6)
- Heptaethylenglycoldibenzoat (R1 = -H, n = 7)
- Octaethylenglycoldibenzoat (R1 = -H, n = 8)
- Nonaethylenglycoldibenzoat (R1 = -H, n = 9)
- Decaethylenglycoldibenzoat (R1 = -H, n = 10)
- Propylenglycoldibenzoat (R1 = -CH₃, n = 1)
- Dipropylenglycoldibenzoat (R1 = -CH₃, n = 2)
- Tripropylenglycoldibenzoat (R1 = -CH₃, n = 3)
- Tetrapropylenglycoldibenzoat (R1 = -CH₃, n = 4)
- Pentapropylenglycoldibenzoat (R1 = -CH₃, n = 5)
- Hexapropylenglycoldibenzoat (R1 = -CH₃, n = 6)
- Heptapropylenglycoldibenzoat (R1 = -CH₃, n = 7)
- Octapropylenglycoldibenzoat (R1 = -CH₃, n = 8)
- Nonapropylenglycoldibenzoat (R1 = -CH₃-CH₃, n = 9)
- Decapropylenglycoldibenzoat (R1 = -CH₃, n = 10)

Ganz besonders bevorzugte erfindungsgemäße Zusammensetzungen sind dadurch gekennzeichnet, dass sie 1,0 bis 10 Gew.-%, weiter bevorzugt 1,25 bis 7,5 Gew.-%, noch weiter bevorzugt 1,5 bis 5 Gew.-% und insbesondere 1,75 bis 3,5 Gew.-% Propylenglycoldibenzoat enthalten.

Zusammenfassend enthalten ganz besonders bevorzugte erfindungsgemäße Zusammensetzungen
- 16,0 Gew.-% PPG-14 Butylether und 1,75 Gew.-% Propylenglycoldibenzoat oder
- 16,0 Gew.-% PPG-14 Butylether und 2,0 Gew.-% Propylenglycoldibenzoat oder
- 16,0 Gew.-% PPG-14 Butylether und 2,25 Gew.-% Propylenglycoldibenzoat oder
- 16,0 Gew.-% PPG-14 Butylether und 2,50 Gew.-% Propylenglycoldibenzoat oder
- 16,0 Gew.-% PPG-14 Butylether und 2,75 Gew.-% Propylenglycoldibenzoat oder
- 16,0 Gew.-% PPG-14 Butylether und 3,0 Gew.-% Propylenglycoldibenzoat oder
- 16,0 Gew.-% PPG-14 Butylether und 3,25 Gew.-% Propylenglycoldibenzoat oder
- 16,0 Gew.-% PPG-14 Butylether und 3,50 Gew.-% Propylenglycoldibenzoat oder
- 16,25 Gew.-% PPG-14 Butylether und 1,75 Gew.-% Propylenglycoldibenzoat oder
- 16,25 Gew.-% PPG-14 Butylether und 2,0 Gew.-% Propylenglycoldibenzoat oder
- 16,25 Gew.-% PPG-14 Butylether und 2,25 Gew.-% Propylenglycoldibenzoat oder
- 16,25 Gew.-% PPG-14 Butylether und 2,50 Gew.-% Propylenglycoldibenzoat oder
- 16,25 Gew.-% PPG-14 Butylether und 2,75 Gew.-% Propylenglycoldibenzoat oder
- 16,25 Gew.-% PPG-14 Butylether und 3,0 Gew.-% Propylenglycoldibenzoat oder
- 16,25 Gew.-% PPG-14 Butylether und 3,25 Gew.-% Propylenglycoldibenzoat oder
- 16,25 Gew.-% PPG-14 Butylether und 3,50 Gew.-% Propylenglycoldibenzoat oder
- 16,5 Gew.-% PPG-14 Butylether und 1,75 Gew.-% Propylenglycoldibenzoat oder
- 16,5 Gew.-% PPG-14 Butylether und 2,0 Gew.-% Propylenglycoldibenzoat oder
- 16,5 Gew.-% PPG-14 Butylether und 2,25 Gew.-% Propylenglycoldibenzoat oder
- 16,5 Gew.-% PPG-14 Butylether und 2,50 Gew.-% Propylenglycoldibenzoat oder
- 16,5 Gew.-% PPG-14 Butylether und 2,75 Gew.-% Propylenglycoldibenzoat oder
- 16,5 Gew.-% PPG-14 Butylether und 3,0 Gew.-% Propylenglycoldibenzoat oder
- 16,5 Gew.-% PPG-14 Butylether und 3,25 Gew.-% Propylenglycoldibenzoat oder
- 16,5 Gew.-% PPG-14 Butylether und 3,50 Gew.-% Propylenglycoldibenzoat oder
- 16,75 Gew.-% PPG-14 Butylether und 1,75 Gew.-% Propylenglycoldibenzoat oder
- 16,75 Gew.-% PPG-14 Butylether und 2,0 Gew.-% Propylenglycoldibenzoat oder
- 16,75 Gew.-% PPG-14 Butylether und 2,25 Gew.-% Propylenglycoldibenzoat oder
- 16,75 Gew.-% PPG-14 Butylether und 2,50 Gew.-% Propylenglycoldibenzoat oder
- 16,75 Gew.-% PPG-14 Butylether und 2,75 Gew.-% Propylenglycoldibenzoat oder
- 16,75 Gew.-% PPG-14 Butylether und 3,0 Gew.-% Propylenglycoldibenzoat oder
- 16,75 Gew.-% PPG-14 Butylether und 3,25 Gew.-% Propylenglycoldibenzoat oder
- 16,75 Gew.-% PPG-14 Butylether und 3,50 Gew.-% Propylenglycoldibenzoat oder
- 17,0 Gew.-% PPG-14 Butylether und 1,75 Gew.-% Propylenglycoldibenzoat oder
- 17,0 Gew.-% PPG-14 Butylether und 2,0 Gew.-% Propylenglycoldibenzoat oder
- 17,0 Gew.-% PPG-14 Butylether und 2,25 Gew.-% Propylenglycoldibenzoat oder
- 17,0 Gew.-% PPG-14 Butylether und 2,50 Gew.-% Propylenglycoldibenzoat oder
- 17,0 Gew.-% PPG-14 Butylether und 2,75 Gew.-% Propylenglycoldibenzoat oder
- 17,0 Gew.-% PPG-14 Butylether und 3,0 Gew.-% Propylenglycoldibenzoat oder
- 17,0 Gew.-% PPG-14 Butylether und 3,25 Gew.-% Propylenglycoldibenzoat oder
- 17,0 Gew.-% PPG-14 Butylether und 3,50 Gew.-% Propylenglycoldibenzoat oder
- 17,25 Gew.-% PPG-14 Butylether und 1,75 Gew.-% Propylenglycoldibenzoat oder
- 17,25 Gew.-% PPG-14 Butylether und 2,0 Gew.-% Propylenglycoldibenzoat oder
- 17,25 Gew.-% PPG-14 Butylether und 2,25 Gew.-% Propylenglycoldibenzoat oder
- 17,25 Gew.-% PPG-14 Butylether und 2,50 Gew.-% Propylenglycoldibenzoat oder
- 17,25 Gew.-% PPG-14 Butylether und 2,75 Gew.-% Propylenglycoldibenzoat oder
- 17,25 Gew.-% PPG-14 Butylether und 3,0 Gew.-% Propylenglycoldibenzoat oder
- 17,25 Gew.-% PPG-14 Butylether und 3,25 Gew.-% Propylenglycoldibenzoat oder
- 17,25 Gew.-% PPG-14 Butylether und 3,50 Gew.-% Propylenglycoldibenzoat oder
- 17,5 Gew.-% PPG-14 Butylether und 1,75 Gew.-% Propylenglycoldibenzoat oder
- 17,5 Gew.-% PPG-14 Butylether und 2,0 Gew.-% Propylenglycoldibenzoat oder
- 17,5 Gew.-% PPG-14 Butylether und 2,25 Gew.-% Propylenglycoldibenzoat oder
- 17,5 Gew.-% PPG-14 Butylether und 2,50 Gew.-% Propylenglycoldibenzoat oder
- 17,5 Gew.-% PPG-14 Butylether und 2,75 Gew.-% Propylenglycoldibenzoat oder
- 17,5 Gew.-% PPG-14 Butylether und 3,0 Gew.-% Propylenglycoldibenzoat oder
- 17,5 Gew.-% PPG-14 Butylether und 3,25 Gew.-% Propylenglycoldibenzoat oder
- 17,5 Gew.-% PPG-14 Butylether und 3,50 Gew.-% Propylenglycoldibenzoat.

Die erfindungsgemäßen Zusammensetzungen können neben den Ölen c1 und c2 weitere Öle enthalten, wobei die Maßgabe, dass der Anteil von Ölkomponenten c) mit einem Brechungsindex ≥ 1,465 weniger als die Hälfte (< 50 Gew.-%) der Gesamtmenge aller Ölkomponenten ausmacht, einzuhalten ist.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 12 - 12000 Pa (0,1 - 90 mm Hg), besonders bevorzugt 13 - 8000 Pa, außerordentlich bevorzugt 30 - 3000 Pa, weiterhin bevorzugt 100 - 400 Pa, aufweisen.

Neben oder an Stelle flüchtiger Siliconöle (s. u.) kann auch mindestens ein flüchtiges Nichtsiliconöl enthalten sein. Bevorzugte flüchtige Nichtsiliconöle sind ausgewählt aus C₈-C₁₆-Isoparaffinen, insbesondere aus Isodecan, Isoundecan, Isododecan, Isotridecan, Isotetradecan, Isopentadecan, und Isohexadecan, sowie Mischungen hiervon. Bevorzugt sind C₁₀-C₁₃-Isoparaffin-Mischungen, insbesondere solche mit einem Dampfdruck bei 20°C von etwa 10 - 400 Pa, bevorzugt 13 - 300 Pa. Dieses mindestens eine flüchtige Nichtsiliconöl, das bevorzugt ausgewählt ist aus C₈-C₁₆-Isoparaffinen, insbesondere aus Isodecan, Isoundecan, Isododecan, Isotridecan, Isotetradecan, Isopentadecan, und Isohexadecan, sowie Mischungen hiervon, ist bevorzugt in einer Gesamtmenge von 10 - 50 Ges.-%, bevorzugt 20 - 40 Gew.-%, besonders bevorzugt 25 - 30 Gew.-%, jeweils bezogen auf die gesamte Zusammensetzung, enthalten.

Aufgrund des trockeneren Hautgefühls und der schnelleren Wirkstofffreisetzung sind als Trägeröl flüchtige Siliconöle, Isoparaffine, insbesondere ausgewählt aus Isodecan, Isoundecan, Isododecan, Isotridecan, Isotetradecan, Isopentadecan, Isohexadecan oder Isoeicosan, sowie Mischungen von flüchtigen Siliconölen und Isoparaffinen, insbesondere ausgewählt aus Isododecan, Isohexadecan und Isoeicosan, besonders bevorzugt.

Erfindungsgemäß bevorzugte kosmetische Öle sind ausgewählt aus flüchtigen Siliconölen, zu denen z. B. Dialkyl- und Alkylarylsiloxane, wie beispielsweise Cyclotetrasiloxan, Cyclopentasiloxan, Cyclohexasiloxan, Dimethylpolysiloxan, niedermolekulares Phenyl Trimethicone und Methylphenylpolysiloxan, aber auch Hexamethyldisiloxan, Octamethyltrisiloxan und Decamethyltetrasiloxan zählen. Besonders bevorzugt sind flüchtige Siliconöle, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten, DC 244, 245, 344 und 345 von Dow Corning (Dampfdruck bei 20°C ca..13 - 15 Pa) enthalten sind.

Erfindungsgemäß besonders bevorzugte Zusammensetzungen sind dadurch gekennzeichnet, dass sie als Ölkomponente c3) 1 bis 40 Gew.-%, vorzugsweise 5 bis 39 Gew.-%, weiter bevorzugt 10 bis 38 Gew.-%, noch weiter bevorzugt 15 bis 37 Gew.-%, weiter bevorzugt 20 bis 36 Gew.-% und insbesondere 25 bis 35 Gew.-% Cyclopentasiloxan enthalten.

Ebenfalls besonders bevorzugt sind flüchtige lineare Siliconöle mit 2 - 10 Siloxaneinheiten, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Ein weiteres bevorzugtes flüchtiges Siliconöl ist ein niedermolekulares Phenyl Trimethicone mit einem Dampfdruck bei 20°C von etwa 2000 Pa, wie es z. B. von GE Bayer Silicones/Momentive unter der Bezeichnung Baysilone Fluid PD 5 erhältlich ist. Flüchtige Siliconöle sind hervorragend geeignete Trägeröle für erfindungsgemäß bevorzugte Antitranspirant-Zusammensetzungen, da sie ihnen ein angenehmes Hautgefühl und eine geringe Kleideranschmutzung verleihen. Erfindungsgemäß besonders bevorzugte Antitranspirant-Zusammensetzungen sind daher durch einen Gehalt an mindestens einem flüchtigen Siliconöl von 10- 95 Gew.-%, bevorzugt 30 - 80 Gew.-%, besonders bevorzugt 40 - 70 Gew.-%, außerordentlich bevorzugt 50 - 60 Gew.-%, jeweils bezogen auf die gesamte Zusammensetzung, gekennzeichnet. Erfindungsgemäß ganz besonders bevorzugte Zusammensetzungen enthalten
- 16,0 Gew.-% PPG-14 Butylether und 1,75 Gew.-% Propylenglycoldibenzoat und 25 bis 35 Gew.-% Cyclopentasiloxan oder
- 16,0 Gew.-% PPG-14 Butylether und 2,0 Gew.-% Propylenglycoldibenzoat und 25 bis 35 Gew.-% Cyclopentasiloxan oder
- 16,0 Gew.-% PPG-14 Butylether und 2,25 Gew.-% Propylenglycoldibenzoat und 25 bis 35 Gew.-% Cyclopentasiloxan oder
- 16,0 Gew.-% PPG-14 Butylether und 2,50 Gew.-% Propylenglycoldibenzoat und 25 bis 35 Gew.-% Cyclopentasiloxan oder
- 16,0 Gew.-% PPG-14 Butylether und 2,75 Gew.-% Propylenglycoldibenzoat und 25 bis 35 Gew.-% Cyclopentasiloxan oder
- 16,0 Gew.-% PPG-14 Butylether und 3,0 Gew.-% Propylenglycoldibenzoat und 25 bis 35 Gew.% Cyclopentasiloxan oder
- 16,0 Gew.-% PPG-14 Butylether und 3,25 Gew.-% Propylenglycoldibenzoat und 25 bis 35 Gew.% Cyclopentasiloxan oder
- 16,0 Gew.-% PPG-14 Butylether und 3,50 Gew.-% Propylenglycoldibenzoat und 25 bis 35 Gew.% Cyclopentasiloxan oder
- 16,25 Gew.-% PPG-14 Butylether und 1,75 Gew.-% Propylenglycoldibenzoat und 25 bis 35 Gew.% Cyclopentasiloxan oder
- 16,25 Gew.-% PPG-14 Butylether und 2,0 Gew.-% Propylenglycoldibenzoat und 25 bis 35 Gew.% Cyclopentasiloxan oder
- 16,25 Gew.-% PPG-14 Butylether und 2,25 Gew.-% Propylenglycoldibenzoat und 25 bis 35 Gew.% Cyclopentasiloxan oder
- 16,25 Gew.-% PPG-14 Butylether und 2,50 Gew.-% Propylenglycoldibenzoat und 25 bis 35 Gew.% Cyclopentasiloxan oder
- 16,25 Gew.-% PPG-14 Butylether und 2,75 Gew.-% Propylenglycoldibenzoat und 25 bis 35 Gew.% Cyclopentasiloxan oder
- 16,25 Gew.-% PPG-14 Butylether und 3,0 Gew.-% Propylenglycoldibenzoat und 25 bis 35 Gew.% Cyclopentasiloxan oder
- 16,25 Gew.-% PPG-14 Butylether und 3,25 Gew.-% Propylenglycoldibenzoat und 25 bis 35 Gew.% Cyclopentasiloxan oder
- 16,25 Gew.-% PPG-14 Butylether und 3,50 Gew.-% Propylenglycoldibenzoat und 25 bis 35 Gew.% Cyclopentasiloxan oder
- 16,5 Gew.-% PPG-14 Butylether und 1,75 Gew.-% Propylenglycoldibenzoat und 25 bis 35 Gew.% Cyclopentasiloxan oder
- 16,5 Gew.-% PPG-14 Butylether und 2,0 Gew.-% Propylenglycoldibenzoat und 25 bis 35 Gew.% Cyclopentasiloxan oder
- 16,5 Gew.-% PPG-14 Butylether und 2,25 Gew.-% Propylenglycoldibenzoat und 25 bis 35 Gew.% Cyclopentasiloxan oder
- 16,5 Gew.-% PPG-14 Butylether und 2,50 Gew.-% Propylenglycoldibenzoat und 25 bis 35 Gew.% Cyclopentasiloxan oder
- 16,5 Gew.-% PPG-14 Butylether und 2,75 Gew.-% Propylenglycoldibenzoat und 25 bis 35 Gew.% Cyclopentasiloxan oder
- 16,5 Gew.-% PPG-14 Butylether und 3,0 Gew.-% Propylenglycoldibenzoat und 25 bis 35 Gew.% Cyclopentasiloxan oder
- 16,5 Gew.-% PPG-14 Butylether und 3,25 Gew.-% Propylenglycoldibenzoat und 25 bis 35 Gew.% Cyclopentasiloxan oder
- 16,5 Gew.-% PPG-14 Butylether und 3,50 Gew.-% Propylenglycoldibenzoat und 25 bis 35 Gew.% Cyclopentasiloxan oder
- 16,75 Gew.-% PPG-14 Butylether und 1,75 Gew.-% Propylenglycoldibenzoat und 25 bis 35 Gew.% Cyclopentasiloxan oder
- 16,75 Gew.-% PPG-14 Butylether und 2,0 Gew.-% Propylenglycoldibenzoat und 25 bis 35 Gew.% Cyclopentasiloxan oder
- 16,75 Gew.-% PPG-14 Butylether und 2,25 Gew.-% Propylenglycoldibenzoat und 25 bis 35 Gew.% Cyclopentasiloxan oder
- 16,75 Gew.-% PPG-14 Butylether und 2,50 Gew.-% Propylenglycoldibenzoat und 25 bis 35 Gew.% Cyclopentasiloxan oder
- 16,75 Gew.-% PPG-14 Butylether und 2,75 Gew.-% Propylenglycoldibenzoat und 25 bis 35 Gew.% Cyclopentasiloxan oder
- 16,75 Gew.-% PPG-14 Butylether und 3,0 Gew.-% Propylenglycoldibenzoat und 25 bis 35 Gew.% Cyclopentasiloxan oder
- 16,75 Gew.-% PPG-14 Butylether und 3,25 Gew.-% Propylenglycoldibenzoat und 25 bis 35 Gew.% Cyclopentasiloxan oder
- 16,75 Gew.-% PPG-14 Butylether und 3,50 Gew.-% Propylenglycoldibenzoat und 25 bis 35 Gew.% Cyclopentasiloxan oder
- 17,0 Gew.-% PPG-14 Butylether und 1,75 Gew.-% Propylenglycoldibenzoat und 25 bis 35 Gew.% Cyclopentasiloxan oder
- 17,0 Gew.-% PPG-14 Butylether und 2,0 Gew.-% Propylenglycoldibenzoat und 25 bis 35 Gew.% Cyclopentasiloxan oder
- 17,0 Gew.-% PPG-14 Butylether und 2,25 Gew.-% Propylenglycoldibenzoat und 25 bis 35 Gew.% Cyclopentasiloxan oder
- 17,0 Gew.-% PPG-14 Butylether und 2,50 Gew.-% Propylenglycoldibenzoat und 25 bis 35 Gew.% Cyclopentasiloxan oder
- 17,0 Gew.-% PPG-14 Butylether und 2,75 Gew.-% Propylenglycoldibenzoat und 25 bis 35 Gew.% Cyclopentasiloxan oder
- 17,0 Gew.-% PPG-14 Butylether und 3,0 Gew.-% Propylenglycoldibenzoat und 25 bis 35 Gew.% Cyclopentasiloxan oder
- 17,0 Gew.-% PPG-14 Butylether und 3,25 Gew.-% Propylenglycoldibenzoat und 25 bis 35 Gew.% Cyclopentasiloxan oder
- 17,0 Gew.-% PPG-14 Butylether und 3,50 Gew.-% Propylenglycoldibenzoat und 25 bis 35 Gew.% Cyclopentasiloxan oder
- 17,25 Gew.-% PPG-14 Butylether und 1,75 Gew.-% Propylenglycoldibenzoat und 25 bis 35 Gew.% Cyclopentasiloxan oder
- 17,25 Gew.-% PPG-14 Butylether und 2,0 Gew.-% Propylenglycoldibenzoat und 25 bis 35 Gew.% Cyclopentasiloxan oder
- 17,25 Gew.-% PPG-14 Butylether und 2,25 Gew.-% Propylenglycoldibenzoat und 25 bis 35 Gew.% Cyclopentasiloxan oder
- 17,25 Gew.-% PPG-14 Butylether und 2,50 Gew.-% Propylenglycoldibenzoat und 25 bis 35 Gew.% Cyclopentasiloxan oder
- 17,25 Gew.-% PPG-14 Butylether und 2,75 Gew.-% Propylenglycoldibenzoat und 25 bis 35 Gew.% Cyclopentasiloxan oder
- 17,25 Gew.-% PPG-14 Butylether und 3,0 Gew.-% Propylenglycoldibenzoat und 25 bis 35 Gew.% Cyclopentasiloxan oder
- 17,25 Gew.-% PPG-14 Butylether und 3,25 Gew.-% Propylenglycoldibenzoat und 25 bis 35 Gew.% Cyclopentasiloxan oder
- 17,25 Gew.-% PPG-14 Butylether und 3,50 Gew.-% Propylenglycoldibenzoat und 25 bis 35 Gew.% Cyclopentasiloxan oder
- 17,5 Gew.-% PPG-14 Butylether und 1,75 Gew.-% Propylenglycoldibenzoat und 25 bis 35 Gew.% Cyclopentasiloxan oder
- 17,5 Gew.-% PPG-14 Butylether und 2,0 Gew.-% Propylenglycoldibenzoat und 25 bis 35 Gew.% Cyclopentasiloxan oder
- 17,5 Gew.-% PPG-14 Butylether und 2,25 Gew.-% Propylenglycoldibenzoat und 25 bis 35 Gew.% Cyclopentasiloxan oder
- 17,5 Gew.-% PPG-14 Butylether und 2,50 Gew.-% Propylenglycoldibenzoat und 25 bis 35 Gew.% Cyclopentasiloxan oder
- 17,5 Gew.-% PPG-14 Butylether und 2,75 Gew.-% Propylenglycoldibenzoat und 25 bis 35 Gew.% Cyclopentasiloxan oder
- 17,5 Gew.-% PPG-14 Butylether und 3,0 Gew.-% Propylenglycoldibenzoat und 25 bis 35 Gew.% Cyclopentasiloxan oder
- 17,5 Gew.-% PPG-14 Butylether und 3,25 Gew.-% Propylenglycoldibenzoat und 25 bis 35 Gew.% Cyclopentasiloxan oder
- 17,5 Gew.-% PPG-14 Butylether und 3,50 Gew.-% Propylenglycoldibenzoat und 25 bis 35 Gew.% Cyclopentasiloxan.

Neben den Ölen c1 und c2 und den vorgenannten, üblicherweise als "flüchtigen" Siliconölen bezeichneten Substanzen sowie neben den vorgenannten flüchtigen Nichtsiliconölen können erfindungsgemäß besonders bevorzugte Antitranspirant-Zusammensetzungen weiterhin mindestens ein nichtflüchtiges kosmetisches Öl, ausgewählt aus nichtflüchtigen Siliconölen und nichtflüchtigen Nichtsiliconölen, enthalten. Durch die relativ schnelle Verdunstung der flüchtigen Öle können feste, unlösliche Bestandteile, insbesondere die Antitranspirantwirkstoffe, auf der Haut als unschöner Rückstand sichtbar werden. Diese Rückstände können erfolgreich mit der erfindungsgemäßen Ölkombination c1 und c2 maskiert werden. Das mindestens eine nichtflüchtige Öl, das von c1 und c2 verschieden ist, kann c1 und c2 darin unterstützen, den negativen Effekt des flüchtigen Öls auf das Rückstandsverhalten erfindungsgemäß bevorzugter Antitranspirant-Zusammensetzungen auszugleichen. Außerdem können mit einem Gemisch aus nichtflüchtigem und flüchtigem Öl Parameter wie Hautgefühl, Sichtbarkeit des Rückstands und Stabilität der Suspension feinreguliert und besser an die Bedürfnisse der Verbraucher angepasst werden.

Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 6 - 50 cSt oder auch 5 - 10 cSt, und Baysilon^{®} 350 M (mit einer kinematischen Viskosität (25°C) von etwa 350 cSt). Erfindungsgemäß ebenfalls bevorzugte Siliconöle sind ausgewählt aus Siliconen der Formel (Sil-1), wobei x ausgewählt ist aus ganzen Zahlen von 1 - 20.

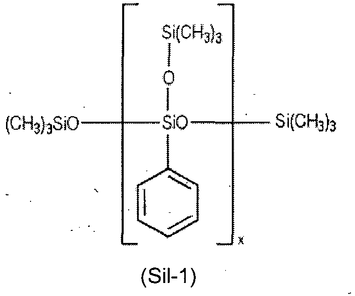

Ein bevorzugtes Siliconöl der Formel (Sil-1) ist unter der INCI-Bezeichnung Phenyl Trimethicone in verschiedenen Qualitäten, Viskositäten und Flüchtigkeiten erhältlich. Ein nicht-flüchtiges Phenyl Trimethicone ist beispielsweise von Dow Corning unter der Bezeichnung Dow Corning 556 erhältlich. Erfindungsgemäß besonders bevorzugte nichtflüchtige Nichtsiliconöle sind ausgewählt aus den Estern der linearen oder verzweigten gesättigten oder nichtflüchtige Nichtsiliconöle ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Hierunter sind Isopropylpalmitat, Isopropylstearat, Isopropylmyristat, 2-Ethylhexylpalmitat (Cegesoft^{®} C 24) und 2-Ethylhexylstearat (Cetiol^{®} 868) außerordentlich bevorzugt. Ebenfalls bevorzugt sind Hexyldecylstearat (Eutanol^{®} G16S), Hexyldecyllaurat, Isononylisononanoat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyl-octanoat, Diisotridecylacetat, n-Hexyllaurat, n-Decyloleat, Oleyloleat, Oleylerucat, Erucyloleat und Erucylerucat.

Ein weiteres erfindungsgemäß bevorzugtes nichtflüchtiges Nichtsiliconöl ist Triethylcitrat.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₅₋₂₂-Alkanole wie Myristylalkohol und Stearylalkohol, die gewünschtenfalls verestert sein können, z. B. PPG-15-Stearylether (Arlamol^{®} E).

Natürliche und synthetische Kohlenwasserstoffe, wie beispielsweise Paraffinöle, C₁₈-C₃₀-Isoparaffine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von BASF), gehören ebenfalls zu den erfindungsgemäß bevorzugten nichtflüchtigen Nichtsiliconölen.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16), Octyldodecanol (Eutanol^{®} G) und 2-Ethylhexylalkohol.

Weitere bevorzugte nichtflüchtige Nichtsiliconöle sind ausgewählt aus Mischungen aus Guerbetalkoholen und Guerbetalkoholestern, z.B. dem Handelsprodukt Cetiol^{®} PGL (Hexyldecanol und Hexyldecyllaurat).

Weitere erfindungsgemäß bevorzugte nichtflüchtige Nichtsiliconöle sind ausgewählt aus den Einfach- und Mehrfachestern von Milchsäure, Citronensäure, Weinsäure oder Adipinsäure mit einem zwei-, drei- oder vierwertigen Alkohol mit 2 bis 9 Kohlenstoffatomen. Besonders bevorzugte Ester dieser Art sind ausgewählt aus Ethylenglycolmonolactat, Ethylenglycolmonocitrat, Ethylenglycolmonotartrat, Ethylenglycolmonoadipat, Ethylenglycoldilactat, Ethylenglycoldicitrat, Ethylenglycolditartrat, Ethylenglycoldiadipat, 1,2-Propylenglycolmonolactat, 1,2-Propylenglycolmonocitrat, 1,2-Propylenglycolmonotartrat, 1,2-Propylenglycolmonoadipat, 1,2-Propylenglycoldilactat, 1,2-Propylenglycoldicitrat, 1,2-Propylenglycolditartrat, 1,2-Propylenglycoldiadipat, 1,3-Propylenglycolmonolactat, 1,3-Propylenglycolmonocitrat, 1,3-Propylenglycolmonotartrat, 1,3-Propylenglycolmonoadipat, 1,3-Propylenglycoldilactat, 1,3-Propylenglycoldicitrat, 1,3-Propylenglycolditartrat, 1,3-Propylenglycoldiadipat, 1,2-Butylenglycolmonolactat, 1,2-Butylenglycolmonocitrat, 1,2-Butylenglycolmonotartrat, 1,2-Butylenglycolmonoadipat, 1,2-Butylenglycoldilactat, 1,2-Butylenglycoldicitrat, 1,2-Butylenglycolditartrat, 1,2-Butylenglycoldiadipat, 1,3-Butylenglycolmonolactat, 1,3-Butylenglycolmonocitrat, 1,3-Butylenglycolmonotartrat, 1,3-Butylenglycolmonoadipat, 1,3-Butylenglycoldilactat, 1,3-Butylenglycoldicitrat, 1,3-Butylenglycolditartrat, 1,3-Butylenglycoldiadipat, 1,4-Butylenglycolmonolactat, 1,4-Butylenglycolmonocitrat, 1,4-Butylenglycolmonotartrat, 1,4-Butylenglycolmonoadipat, 1,4-Butylenglycoldilactat, 1,4-Butylenglycoldicitrat, 1,4-Butylenglycolditartrat, 1,4-Butylenglycoldiadipat, 1,2-Pentandiolmonolactat, 1,2-Pentandiolmonocitrat, 1,2-Pentandiolmonotartrat, 1,2-Pentandiolmonoadipat, 1,2-Pentandioldilactat, 1,2-Pentandioldicitrat, 1,2-Pentandiolditartrat, 1,2-Pentandioldiadipat, 1,3-Pentandiolmonolactat, 1,3-Pentandiolmonocitrat, 1,3-Pentandiolmonotartrat, 1,3-Pentandiolmonoadipat, 1,3-Pentandioldilactat, 1,3-Pentandioldicitrat, 1,3-Pentandiolditartrat, 1,3-Pentandioldiadipat, 1,4-Pentandiolmonolactat, 1,4-Pentandiolmonocitrat, 1,4-Pentandiolmonotartrat, 1,4-Pentandiolmonoadipat, 1,4-Pentandioldilactat, 1,4-Pentandioldicitrat, 1,4-Pentandiolditartrat, 1,4-Pentandioldiadipat, 1,5-Pentandiolmonolactat, 1,5-Pentadiolmonocitrat, 1,5-Pentandiolmonotartrat, 1,5-Pentandiolmonoadipat, 1,5-Pentandioldilactat, 1,5-Pentandioldicitrat, 1,5-Pentandiolditartrat, 1,5-Pentandioldiadipat, 1,2-Hexandiolmonolactat, 1,2-Hexandiolmonocitrat, 1,2-Hexandiolmonotartrat, 1,2-Hexandiolmonoadipat, 1,2-Hexandioldilactat, 1,2-Hexandioldicitrat, 1,2-Hexandiolditartrat, 1,2-Hexandioldiadipat, 1,3-Hexandiolmonolactat, 1,3-Hexandiolmonocitrat, 1,3-Hexandiolmonotartrat, 1,3-Hexandiolmonoadipat, 1,3-Hexandioldilactat, 1,3-Hexandioldicitrat, 1,3-Hexandiolditartrat, 1,3-Hexandioldiadipat, 1,4-Hexandiolmonolactat, 1,4-Hexandiolmonocitrat, 1,4-Hexandiolmonotartrat, 1,4-Hexandiolmonoadipat, 1,4-Hexandioldilactat, 1,4-Hexandioldicitrat, 1,4-Hexandiolditartrat, 1,4-Hexandioldiadipat, 1,5-Hexandiolmonolactat, 1,5-Hexandiolmonocitrat, 1,5-Hexandiolmonotartrat, 1,5-Hexandiolmonoadipat, 1,5-Hexandioldilactat, 1,5-Hexandioldicitrat, 1,5-Hexandiolditartrat, 1,5-Hexandioldiadipat, 1,6-Hexandiolmonölactat, 1,6-Hexandiolmonocitrat, 1,6-Hexandiolmonotartrat, 1,6-Hexandiolmonoadipat, 1,6-Hexandioldilactat, 1,6-Hexandioldicitrat, 1,6-Hexandiolditartrat, 1,6-Hexandioldiadipat, 2-Ethylhexan-1,2-diolmonolactat, 2-Ethylhexan-1,2-diolmonocitrat, 2-Ethylhexan-1,2-diolmonotartrat, 2-Ethylhexan-1,2-diolmono-adipat, 2-Ethylhexan-1,2-dioldilactat, 2-Ethylhexan-1,2-dioldicitrat, 2-Ethylhexan-1,2-diolditartrat, 2-Ethylhexan-1,2-dioldiadipat, 2-Ethylhexan-1,3-diolmonolactat, 2-Ethylhexan-1,3-diolmonocitrat, 2-Ethylhexan-1,3-diolmonotartrat, 2-Ethylhexan-1,3-diolmonoadipat, 2-Ethylhexan-1,3-dioldilactat, 2-Ethylhexan-1,3-dioldicitrat, 2-Ethylhexan-1,3-diolditartrat, 2-Ethylhexan-1,3-dioldiadipat, 2-Ethylhexan-1,4-diolmonolactat, 2-Ethylhexan-1,4-diolmonocitrat, 2-Ethylhexan-1,4-diolmonotartrat, 2-Ethylhexan-1,4-diolmonoadipat, 2-Ethylhexan-1,4-dioldilactat, 2-Ethylhexan-1,4-dioldicitrat, 2-Ethylhexan-1,4-diolditartrat, 2-Ethylhexan-1,4-dioldiadipat, 2-Ethylhexan-1,5-diolmonolactat, 2-Ethylhexan-1,5-diolmonocitrat, 2-Ethylhexan-1,5-diolmonotartrat, 2-Ethylhexan-1,5-diolmonoadipat, 2-Ethylhexan-1,5-dioldilactat, 2-Ethylhexan-1,5-dioldicitrat, 2-Ethylhexan-1,5-diolditartrat, 2-Ethylhexan-1,5-dioldiadipat, 2-Ethylhexan-1,6-diolmonolactat, 2-Ethylhexan-1,6-diolmonocitrat, 2-Ethylhexan-1,6-diolmonotartrat, 2-Ethylhexan-1,6-diolmonoadipat, 2-Ethylhexan-1,6-dioldilactat, 2-Ethylhexan-1,6-dioldicitrat, 2-Ethylhexan-1,6-diolditartrat, 2-Ethylhexan-1,6-dioldiadipat, 1,2-Heptandiolmonolactat, 1,2-Heptandiolmonocitrat, 1,2-Heptandiolmonotartrat, 1,2-Heptandiolmonoadipat, 1,2-Heptandioldilactat, 1,2-Heptandioldicitrat, 1,2-Heptandiolditartrat, 1,2-Heptandioldiadipat, 1,3-Heptandiolmonolactat, 1,3-Heptandiolmonocitrat, 1,3-Heptandiolmonotartrat, 1,3-Heptandiolmonoadipat, 1,3-Heptandioldilactat, 1,3-Heptandioldicitrat, 1,3-Heptandiolditartrat, 1,3-Heptandioldiadipat, 1,4-Heptandiolmonolactat, 1,4-Heptandiolmonocitrat, 1,4-Heptandiolmonotartrat, 1,4-Heptandiolmonoadipat, 1,4-Heptandioldilactat, 1,4-Heptandioldicitrat, 1,4-Heptandiolditartrat, 1,4-Heptandioldiadipat, 1,5-Heptandiolmonolactat, 1,5-Heptandiolmonocitrat, 1,5-Heptandiolmonotartrat, 1,5-Heptandiolmonoadipat, 1,5-Heptandioldilactat, 1,5-Heptandioldicitrat, 1,5-Heptandiolditartrat, 1,5-Heptandioldiadipat, 1,6-Heptandiolmonolactat, 1,6-Heptandiolmonocitrat, 1,6-Heptandiolmonotartrat, 1,6-Heptandiolmonoadipat, 1,6-Heptandioldilactat, 1,6-Heptandioldicitrat, 1,6-Heptandiolditartrat, 1,6-Heptandioldiadipat, 1,7-Heptandiolmonolactat, 1,7-Heptandiolmonocitrat, 1,7-Heptandiolmonotartrat, 1,7-Heptandiolmonoadipat, 1,7-Heptandioldilactat, 1,7-Heptandioldicitrat, 1,7-Heptandiolditartrat, 1,7-Heptandioldiadipat, 1,2-Octandiolmonolactat, 1,2-Octandiolmonocitrat, 1,2-Octandiolmonotartrat, 1,2-Octandiolmonoadipat, 1,2-Octandioldilactat, 1,2-Octandioldicitrat, 1,2-Octandiolditartrat, 1,2-Octandioldiadipat, 1,3-Octandiolmonolactat, 1,3-Octandiolmonocitrat, 1,3-Octandiolmonotartrat, 1,3-Octandiolmonoadipat, 1,3-Octandioldilactat, 1,3-Octandioldicitrat, 1,3-Octandiolditartrat, 1,3-Octandioldiadipat, 1,4-Octandiolmonolactat, 1,4-Octandiolmonocitrat, 1,4-Octandiolmonotartrat, 1,4-Octandiolmonoadipat, 1,4-Octandioldilactat, 1,4-Octandioldicitrat, 1,4-Octandiolditartrat, 1,4-Octandioldiadipat, 1,5-Octandiolmonolactat, 1,5-Octandiolmonocitrat, 1,5-Octandiolmonotartrat, 1,5-Octandiolmonoadipat, 1,5-Octandioldilactat, 1,5-Octandioldicitrat, 1,5-Octandiolditartrat, 1,5-Octandioldiadipat, 1,6-Octandiolmonolactat, 1,6-Octandiolmonocitrat, 1,6-Octandiolmonotartrat, 1,6-Octandiolmonoadipat, 1,6-Octandioldilactat, 1,6-Octandioldicitrat, 1,6-Octandiolditartrat, 1,6-Octandioldiadipat, 1,7-Octandiolmonolactat, 1,7-Octandiolmonocitrat, 1,7-Octandiolmonotartrat, 1,7-Octandiolmonoadipat, 1,7-Octandioldilactat, 1,7-Octandioldicitrat, 1,7-Octandiolditartrat, 1,7-Octandioldiadipat, 1,8-Octandiolmonolactat, 1,8-Octandiolmonocitrat, 1,8-Octandiolmonotartrat, 1,8-Octandiolmonoadipat, 1,8-Octandioldilactat, 1,8-Octandioldicitrat, 1,8-Octandiolditartrat, 1,8-Octandioldiadipat, 2-Methyl-1,3-propandiolmonolactat, 2-Methyl-1,3-propandiolmonocitrat, 2-Methyl-1,3-propandiolmonotartrat, 2-Methyl-1,3-propandiolmonoadipat, 2-Methyl-1,3-propandioldilactat, 2-Methyl-1,3-propandioldicitrat, 2-Methyl-1,3-propandiolditartrat, 2-Methyl-1,3-propandioldiadipat, Dipropylenglycolmonolactat, Dipropylenglycolmonotartrat, Dipropylenglycolmonocitrat, Dipropylenglycolmonoadipat, Dipropylenglycoldilactat, Dipropylenglycolditartrat, Dipropylenglycoldicitrat, Dipropylenglycoldiadipat, Glycerinmonolactat, Glycerinmonotartrat, Glycerinmonocitrat, Glycerinmonoadipat, Glycerindilactat, Glycerinditartrat, Glycerindicitrat, Glycerindiadipat, Glycerintrilactat, Glycerintritartrat, Glycerintricitrat, Glycerintriadipat, Diglycerinmonolactat, Diglycerinmonotartrat, Diglycerinmonocitrat, Diglycerinmonoadipat, Diglycerindilactat, Diglycerinditartrat, Diglycerindicitrat, Diglycerindiadipat, Diglycerintrilactat, Diglycerintritartrat, Diglycerintricitrat, Diglycerintriadipat, Tripropylenglycolmonolactat, Tripropylenglycolmonotartrat, Tripropylenglycolmonocitrat, Tripropylenglycolmonoadipat, Tripropylenglycoldilactat, Tripropylenglycolditartrat, Tripropylenglycoldicitrat, Tripropylenglycoldiadipat, Tripropylenglycoltrilactat, Tripropylenglycoltritartrat, Tripropylenglycoltricitrat, Tripropylenglycoltriadipat, Triglycerinmonolactat, Triglycerinmonotartrat, Triglycerinmonocitrat, Triglycerinmonoadipat, Triglycerindilactat, Triglycerinditartrat, Triglycerindicitrat, Triglycerindiadipat, Triglycerintrilactat, Triglycerintritartrat, Triglycerintricitrat, Triglycerintriadipat, 1,2,6-Hexantriolmonolactat, 1,2,6-Hexantriolmonotartrat, 1,2,6-Hexantriolmonocitrat, 1,2,6-Hexantriolmonoadipat, 1,2,6-Hexantrioldilactat, 1,2,6-Hexantriolditartrat, 1,2,6-Hexantrioldicitrat, 1,2,6-Hexantrioldiadipat, 1,2,6-Hexantrioltrilactat, 1,2,6-Hexantrioltritartrat, 1,2,6-Hexantrioltricitrat, 1,2,6-Hexantrioltriadipat, Trimethylolpropanmonolactat, Trimethylolpropanmonotartrat, Trimethylolpropanmonocitrat, Trimethylolpropanmonoadipat, Trimethylolpropandilactat, Trimethylolpropanditartrat, Trimethylolpropandicitrat, Trimethylolpropandiadipat, Trimethylolpropantrilactat, Trimethylolpropantritartrat, Trimethylolpropantricitrat, Trimethylolpropantriadipat, Trimethylolethanmonolactat, Trimethylolethanmonotartrat, Trimethylolethanmonocitrat, Trimethylolethanmonoadipat, Trimethylolethandilactat, Trimethylolethanditartrat, Trimethylolethandicitrat, Trimethylolethandiadipat, Trimethylolethantrilactat, Trimethylolethantritartrat, Trimethylolethantricitrat und Trimethylolethantriadipat, sowie Mischungen hiervon.

Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (BASF) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (BASF) mit verzweigten Fettsäureresten.

Weitere erfindungsgemäß besonders bevorzugte nichtflüchtige Nichtsiliconöle sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC), Di-n-octylcarbonat, Di-n-dodecylcarbonat, Di-(2-ethylhexyl)carbonat oder die Ester gemäß der Lehre der DE 19756454 A.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Unabhängig davon, ob über die Öle c1 und c2 hinaus weitere Öle in den erfindungsgemäßen Zur sammensetzungen enthalten sind, sind Zusammensetzung bevorzugt, bei denen das Gewichtsverhältnis von c1) zu c2) 1 zu 1 bis 20 zu 1, vorzugsweise 1 zu 1 bis 10 zu 1, weiter bevorzugt 1 zu 1 bis 5 zu 1, noch weiter bevorzugt 1 zu 1 bis 2 zu 1 und insbesondere 1 zu 1 bis 1,5 zu 1 beträgt.

Ebenfalls unabhängig davon, ob über die Öle c1 und c2 hinaus weitere Öle in den erfindungsgemäßen Zusammensetzungen enthalten sind, sind Zusammensetzung bevorzugt, bei denen der Anteil von Ölkomponenten c) mit einem Brechungsindex ≥ 1,465 weniger als 45 Gew.-%, vorzugsweise weniger als 40 Gew.-% des Gesamtgewichts aller Ölkomponenten ausmacht.

Duft- und Riechstoffe zählen erfindungsgemäß nicht zu den Ölen, die bei der Berechnung des Gewichtsanteils der vorstehend beschriebenen Trägeröle c) berücksichtigt werden. Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems. Beispiele für Duft- und Riechstoffverbindungen vom Typ der Ester sind Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Beispiele für Duft- und Riechstoffverbindungen vom Typ der Ether sind Benzylethylether und Ambroxan, Beispiele für Duft- und Riechstoffverbindungen vom Typ der Aldehyde sind die linearen Alkanale mit 8 - 18 C-Atomen, Citral, Citronellal, Citronellyloxy-acetaldehyd, Cyclamenaldehyd, Lilial und Bourgeonal, Beispiele für Duft- und Riechstoffverbindungen vom Typ der Ketone sind die Jonone, alpha-Isomethylionon und Methylcedrylketon, Beispiele für Duft- und Riechstoffverbindungen vom Typ der Alkohole sind Anethol, Citronellol, Eugenol, Geraniol, Linalool, Phenylethylalkohol und Terpineol, Beispiele für Duft- und Riechstoffverbindungen vom Typ der Terpene sind Limonen und Pinen. Beispiele für Duft- und Riechstoffverbindungen sind Pine-, Citrus-, Jasmin-, Patchouly-, Rosen-, Ylang-Ylang-Öl, Muskateller-Salbeiöl, Kamillenöl, Nelkenöl, Minzöl, Zimtblätteröl, Lindenblütenöl, Wacholderbeeröl, Vetiveröl, Olibanumöl, Galbanumöl, Labdanumöl, Orangenblütenöl, Neroliöl, Orangenschalenöl und Sandelholzöl, weiterhin die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Niaouliöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Wacholderbeeröl, Wermutöl, Wintergrünöl, Ysop-Öl, Zimtöl, Zitronellöl, Zitronenöl und Zypressenöl. Weitere Duft- und Riechstoffverbindungen sind Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonyl-acetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadecanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undecalacton, Vanillin, Veratrumaldehyd, Zimtaldehyd, Zimatalkohol, Zimtsäure, Zimtsäureethylester und Zimtsäurebenzylester.

Weitere (leichter flüchtige) Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral und Zitronellal.

Bevorzugt werden Mischungen verschiedener Duftstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Arömakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.

Bevorzugte erfindungsgemäße Zusammensetzungen sind dadurch gekennzeichnet, dass mindestens ein Duftstoff in einer Gesamtmenge von 0,1 - 15 Gew.-%, bevorzugt 0,5-10 Gew.-%, besonders bevorzugt 1 - 8 Gew.-%, außerordentlich bevorzugt 2 - 7 Gew.-%, weiterhin außerordentlich bevorzugt 3 - 6 Gew.-%, jeweils bezogen auf das Gesamtgewicht der treibmittelfreien Zusammensetzung, enthalten ist.

Weitere erfindungsgemäß bevorzugte Zusammensetzungen sind gekennzeichnet durch einen Gehalt an mindestens einem so genannten "hautkühlenden Wirkstoff'. Unter hautkühlenden Wirkstoffen im Sinne der vorliegenden Anmeldung werden Wirkstoffe verstanden, die bei Applikation auf die Haut infolge Oberflächenanästhesierung und Reizung der kälteempfindlichen Nerven bei Migräne und dergleichen ein angenehmes Kältegefühl erzeugen, auch wenn die behandelten Hautpartien tatsächlich normale bzw. erhöhte Temperatur zeigen. Als Haut kühlende Wirkstoffe sind erfindungsgemäß solche Verbindungen anzusehen, die, ähnlich wie I-Menthol, die Thermorezeptoren in der Haut und den Schleimhäuten so stimulieren, dass ein kühler sensorischer Eindruck entsteht. Insbesondere der Rezeptor CMR-1 ("cold- and menthol-sensitive receptor"), der zur Familie der TRP-Kanäle gehört, wird durch die Kühlwirkstoffe stimuliert, wodurch ein Kälteeindruck erzeugt wird.

Bevorzugte hautkühlende Wirkstoffe sind insbesondere Menthol, Isopulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Als hautkühlende Wirkstoffe besonders bevorzugt sind Menthol, Isopulegol, Menthyllactat, Menthoxypropandiol und Menthylpyrrolidoncarbonsäure.

Bevorzugte erfindungsgemäße Zusammensetzungen enthalten mindestens einen hautkühlenden Wirkstoff in Gesamtmengen von 0,01 - 1 Gew.-%, bevorzugt 0,02 - 0,5 Gew.-% und besonders bevorzugt 0,05 - 0,2 Gew.-%, jeweils bezogen auf das Gesamtgewicht der (treibmittelfreien) Zusammensetzung.

Bevorzugte erfindungsgemäße Zusammensetzungen sind dadurch gekennzeichnet, dass mindestens ein verkapselter Wirkstoff enthalten ist. Die Wirkstoffe, die vorteilhafterweise verkapselt sein können, sind insbesondere Duftstoffe, Parfümöle und/oder hautkühlende Wirkstoffe, aber auch andere hautpflegende Wirkstoffe, wie Vitamine, Antioxidantien etc.

Als Kapselmaterial bevorzugt sind wasserlösliche Polymere wie Stärke, physikalisch und/oder chemisch modifizierte Stärken, Cellulosederivate, wie z. B. Carboxymethylcellulose, Methylcellulose, Hydroxyethylcellulose oder Hydroxypropylmethylcellulose, Carragheene, Alginate, Maltodextrine, Dextrine, Pflanzengummen, Pektine, Xanthane, Polyvinylacetat und Polyvinylalkohol, Polyvinylpyrrolidin, Polyamide, Polyester und Homo- und Copolymere aus Monomeren, ausgewählt aus Acrylsäure, Methacrylsäure, Maleinsäure, Fumarsäure, Itaconsäure sowie den Estern und den Salzen dieser Säuren, sowie beliebige Mischungen dieser Polymeren.

Bevorzugte Kapselmaterialien sind chemisch modifizierte Stärken, insbesondere Aluminiumstärkeoctenylsuccinat, z. B. das Handelsprodukt Dry Flo Plus von National Starch, oder Natriumstärkeoctenylsuccinat, z. B. das Handelsprodukt Capsul von National Starch, des weiteren Carboxymethylcellulose, Carboxymethylcellulose, Methylcellulose, Hydroxyethylcellulose und Hydroxypropylmethylcellulose, Ethylcellulose, z. B. das Handelsprodukt Tylose H 10 von Clariant, weiterhin Carragheene, Alginate und Maltodextrine, sowie beliebige Mischungen dieser Polymere.

In einer weiteren erfindungsgemäß bevorzugten Ausführungsform enthalten die erfindungsgemäßen Zusammensetzungen 0 bis maximal 5 Gew.-% Ethanol.

Weiterhin können die erfindungsgemäßen Zusammensetzungen zusätzliche Deodorant-Wirkstoffe enthalten. Als Deodorant-Wirkstoffe können antimikrobielle, antibakterielle oder keimhemmende Stoffe, Antioxidantien oder Geruchsadsorbentien (z. B. Zinkricinoleat) eingesetzt werden. Geeignete antimikrobielle, antibakterielle oder keimhemmende Stoffe sind insbesondere Organohalogenverbindungen sowie -halogenide, quartäre Ammoniumverbindungen, eine Reihe von Pflarizenextrakten und Zinkverbindungen. Bevorzugt sind halogenierte Phenolderivate wie z. B. Hexachlorophen oder Irgasan DP 300 (Triclosan, 2,4,4'-Trichlor-2'-hydroxydiphenylether), 3,4,4'-Trichlorcarbonilid, Chlorhexidin (1,1'-Hexamethylen-bis-[5-(4-chlorphenyl)]-biguanid), Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Auch Benzylalkohol kann als Deodorant-Wirkstoff eingesetzt werden. Weitere antibakteriell wirksame Deodorantien sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide. Bevorzugte Deodorant-Wirkstoffe sind langkettige Diole, z. B. 1,2-Alkan-(C₅-C₁₈)-Diole, Glycerinmono(C₈-C₁₈)-Fettsäureester oder, besonders bevorzugt, Glycerinmono-(C₆-C₁₆)-alkylether, insbesondere 2-Ethylhexylglycerinether, die sehr gut haut- und schleimhautverträglich und gegen Corynebakterien wirksam sind, sowie weiterhin Phenoxyethanol, Phenoxyisopropanol (3-Phenoxy-propan-2-ol), Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenyl-butan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-1-ol, 2-Ethyl-3-phenylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethyl-propan-1-ol, 2-Allyl-3-phenylpropan-1-ol und 2-n-Pentyl-3-phenylpropan-1-ol.

Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind z. B. die Salze der Ethylendiamintetraessigsäure oder der Nitrilotriessigsäure sowie die Salze der 1-Hydroxyethan-1,1-diphosphonsäure.

Die Konfektionierung der erfindungsgemäßen Zusammensetzungen, die als Spray appliziert werden, richtet sich vorzugsweise nach den Anforderungen der gewünschten Sprayapplikation.

Die erfindungsgemäßen Zusammensetzungen liegen als Suspension vor, das heißt, der schweißhemmende Wirkstoff und gegebenenfalls weitere unlösliche Bestandteile sind in einem flüssigen oder festen Träger suspendiert. Flüssig-disperse Systeme dieser Art, z. B. Rollons oder als Spray zu applizierende Dispersionen, sollten vor der Anwendung geschüttelt werden.

In einer weiteren erfindungsgemäß bevorzugten Ausführungsform sind die erfindungsgemäßen Zusammensetzungen als mit einem Treibmittel versprühbare Suspension konfektioniert. Bevorzugte erfindungsgemäße Zusammensetzungen können z. B. in Pump- oder Quetschspendern abgepackt sein, insbesondere in Mehrkammer-Pump- oder Quetschspendern. Derartige Spender verwenden Luft, insbesondere die Umgebungsluft, als Treibmittel bzw. fördern die erfindungsgemäße Zusammensetzung durch Pumpen.

In einer weiteren bevorzugten Ausführungsform der Erfindung wird die Zusammensetzung mittels eines komprimierten bzw. verflüssigten Treibmittels appliziert.

Alle Mengenangaben beziehen sich, sofern nicht anders angegeben, auf das Gewicht der treibmittelfreien Zusammensetzung.

Die Verpackung in einem Mehrkammerspender bietet besondere technische Vorteile.

Der Mehrkammerspender kann auch so eingesetzt werden, dass eine Kammer mit der erfindungsgemäßen Zusammensetzung befüllt ist, während eine andere Kammer das komprimierte Treibmittel enthält. Ein derartiger Mehrkammerspender ist beispielsweise eine so genannte Bag-in-Can-Verpackung.

Beide Kammern können aber auch so miteinander verbunden, dass die erfindungsgemäße Zusammensetzung in zwei Teilzusammensetzungen aufgetrennt wird, die gleichzeitig aus der Verpackung ausgegeben werden können, beispielsweise aus getrennten Öffnungen oder aus einer einzigen Öffnung.

Weitere bevorzugte erfindungsgemäße Zusammensetzungen sind dadurch gekennzeichnet, dass sie mit mindestens einem Treibmittel in einem geeigneten Druckbehälter verpackt sind. Erfindungsgemäß bevorzugte Treibmittel (Treibgase) sind ausgewählt aus Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1, 1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination.

Besonders bevorzugt sind Propan, n-Butan, iso-Butan sowie, besonders bevorzugt, Mischungen dieser Treibmittel. Weiterhin bevorzugt sind auch 1,1-Difluorethan, Propan, n-Butan, iso-Butan sowie Mischungen dieser Treibmittel, insbesondere Mischungen aus 1,1-Difluorethan und n-Butan.

Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.

Die Menge der Treibmittel beträgt bevorzugt 10 - 95 Gew.-%, besonders bevorzugt 30 - 90 Gew.-% und außerordentlich bevorzugt 60 - 86 Gew.-%, und weiterhin außerordentlich bevorzugt 70, 72, 74, 76, 78, 82, 84 oder 85 Gew.-%, jeweils bezogen auf das Gesamtgewicht der Zubereitung, bestehend aus der erfindungsgemäßen Suspension und dem Treibmittel.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der erfindungsgemäßen Suspension. Ein erfindungsgemäß bevorzugter Innenschutzlack ist ein Epoxy-Phenollack, wie er u.a. unter der Bezeichnung Hoba 7407 P erhältlich ist. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein.

Die Dosen sind mit einem geeigneten Sprühkopf ausgestattet. Je nach Sprühkopf sind Ausstoßraten, bezogen auf voll gefüllte Dosen, von 0,1 g/s bis 2,0 g/s möglich.

Ein weiterer Gegenstand der vorliegenden Anmeldung ist ein nicht-therapeutisches, kosmetisches Verfahren zur Reduzierung und/oder Regulierung der Schweißbildung und/oder des Körpergeruchs, bei dem eine Zusammensetzung nach einem der Ansprüche 1 bis 9 in einer wirksamen Menge auf die Haut, bevorzugt auf die Haut im Achselbereich, aufgetragen wird.

Bezüglich weiterer bevorzugter Ausführungsformen des erfindungsgemäßen Verfahrens gilt mutatis mutandis das zu den erfindungsgemäßen Zusammensetzungen Gesagte.

Ein weiterer Gegenstand der vorliegenden Anmeldung ist die Verwendung von Mischungen aus
c1) PPG-14 Butylether und
c2) mindestens einem Öl aus der Gruppe der Alkylbenzoate, der Alkylendibenzoate, der alkoxylierten Alkylbenzoate, der Polyalkylenoxid-Dibenzoate oder deren Mischungen mit der Maßgabe, dass der Gewichts-Anteil von Ölkomponenten mit einem Brechungsindex ≥ 1,465 weniger als die Hälfte des Gesamtgewichts der Mischung ausmacht, und weiterhin mit der Maßgabe, dass die Zusammensetzung - jeweils bezogen auf ihr Gewicht ggf. nach Abzug flüchtiger Treibmittel - 0,75 bis 15 Gew.-% Propylenglycoldibenzoat enthält,
zur Verhinderung oder Verringerung sichtbarer Rückstände von Antitranspirant-Zusammensetzungen. Bezüglich weiterer bevorzugter Ausführungsformen der erfindungsgemäßen Verwendungen gilt mutatis mutandis das zu den erfindungsgemäßen Zusammensetzungen Gesagte.

### Beispiele:

Alle Angaben in Gew.-%.

Es wurden folgende Zusammensetzungen hergestellt:

| | V1 | V2 | V3 | E | V4 | V5 |
|---|---|---|---|---|---|---|
| | Gew.-% | Gew.-% | Gew.-% | Gew.-% | Gew.-% | Gew.-% |
| Aluminum Chorohydrat | 20,0 | 20,0 | 20,0 | 20,0 | 20,0 | 20,0 |
| PPG-14 Butyl Ether | - | 9,5 | - | 9,5 | 19,0 | - |
| Propylene Glycol Dibenzoate | - | - | 9,5 | 9,5 | - | 19,0 |
| Talc | 3,1 | 3,1 | 3,1 | 3,1 | 3,1 | 3,1 |
| Stearyl Alcohol | 17,4 | 17,4 | 17,4 | 17,4 | 17,4 | 17,4 |
| Cocoglyceride | 4,0 | 4,0 | 4,0 | 4,0 | 4,0 | 4,0 |
| Hydrogeniertes Rizinusöl | 1,5 | 1,5 | 1,5 | 1,5 | 1,5 | 1,5 |
| Cyclopentasiloxan | 51,0 | 41,5 | 41,5 | 32,0 | 32,0 | 32,0 |
| Ceteareth 30 | 3,0 | 3,0 | 3,0 | 3,0 | 3,0 | 3,0 |
| | | | | | | |
| Mittlerer Score (N = 8) | 2,9 | 2,5 | 1,9 | 0,5 | 2,1 | 0,8 |
| Standardabweichung | 0,2 | 0,4 | 0,6 | 0,2 | 0,5 | 0,2 |
| Differenz zu Rezeptur V1 | | **-0,4** | **-0,9** | **-2,4** | **-0,8** | **-2,1** |

Die Antitranspirant-Stift-Rezepturen V1 bis V5 und E wurden bei konstantem Druck auf schwarze Pappe ausgestrichen. Der entstandene weiße Rückstand wurde von 8 Testpersonen im Vergleich zu Referenzscores visuell bewertet. Ein Wert von 3 entspricht dabei dem maximalen Rückstand und ein Wert von 0 der Abwesenheit von Rückstand.

Bei einem rein additiven Effekt durch Kombination von 9,5% PPG-14 Butyl Ether mit 9,5% Propylene Glycol Dibenzoate wäre eine Reduktion des Rückstandes um -1,3 Einheiten zu erwarten (Rezeptur V2: -0,4; Rezeptur V3: -0,9). Tatsächlich reduziert die Rezeptur E den Rückstand um 2,4 Einheiten, so dass ein synergistischer Effekt von PPG-14 Butyl Ether und Propylene Glycol Dibenzoate nachgewiesen werden konnte. Auch bei Einsatz von 19,0% Propylene Glycol Dibenzoate (Rezeptur V5) wird die Wirkung der Kombination nicht erreicht.

Durch Kombination von PPG-14 Butyl Ether mit Propylene Glycol Dibenzoate kommt es zu einer synergistischen Verminderung weißer Rückstände.

Weitere Rezepturbeispiele für Antitranspirant-Stifte

| | 1 | 2 | 3 |
|---|---|---|---|
| | Gew.-% | Gew.-% | Gew.-% |
| Aluminum Chorohydrat (Microdry 3115 von Summit Reheis) | - | 22,0 | - |
| Aluminum Zirconium Pentachorohydrex Gly (AAZG 3110 von Summit Reheis) | 22,0 | -- | - |
| Aluminum Zirconium Trichorohydrex Gly (AAZG 531 von Summit Reheis) | - | - | 17,8 |
| PPG-14 Butyl Ether (Ucon Fluid AP von DOW) | 9,5 | 9,5 | 9,5 |
| Propylene Glycol Dibenzoate (Lexfeel Shine von Inolex Chemical Company) | 9,5 | 9,5 | 9,5 |
| Talc | | | 3,0 |
| Stearyl Alcohol, (Lanette 18 von BASF) | 20,0 | 20,0 | 19,0 |
| Cocoglyceride (Novata AB PH von BASF) | | | |
| Hydrogeniertes Rizinusöl (Cutina HR von BASF) | 3,0 | 3,0 | 4,8 |
| Cyclopentasiloxan | 33,0 | 33,0 | 29,7 |
| Ceteareth 30 (Eumulgin B3 von BASF) | | | |
| Myristyl Myristat (Crodamol MM) | 2,0 | 2,0 | 2,0 |
| Cetylalkohol | | | 2,5 |
| Performalene 500 Polyethylene | | | 1,2 |
| Parfum | 1,0 | 1,0 | 1,0 |

## Patentansprüche

1. Schweißhemmende Zusammensetzung zur persönlichen Körperpflege, konfektioniert als Nonaerosol, Stift, Soft Solid, Creme, Gel, nicht-versprühbare oder versprühbare Suspension, nicht-versprühbare Lösung oder auf einem Substrat imprägniert, enthaltend - jeweils bezogen auf ihr Gewicht ggf. nach Abzug flüchtiger Treibmittel -
a) mindestens einen schweißhemmenden Wirkstoff,
b) 0 - 7 Gew.-%, bevorzugt 0 - 3 Gew.-%, freies Wasser,
c) eine Mischung mindestens zweier unter Normalbedingungen flüssiger Öle als Träger,
wobei die Zusammensetzung
c1) 5 bis 30 Gew.-% PPG-14 Butylether und
c2) 0,5 bis 20 Gew.-% mindestens eines Öls aus der Gruppe der Alkylbenzoate, der Alkylendibenzoate, der alkoxylierten Alkylbenzoate, der Polyalkylenoxid-Dibenzoate oder deren Mischungen
enthält, mit der Maßgabe, dass der Gewichts-Anteil von Ölkomponenten mit einem Brechungsindex ≥ 1,465 weniger als die Hälfte des Gesamtgewichts aller Ölkomponenten c) ausmacht,
und weiterhin mit der Maßgabe, dass die Zusammensetzung 0,75 bis 15 Gew.-%, Propylenglycoldibenzoat enthält.

2. Zusammensetzung nach Anspruch 1, **dadurch gekennzeichnet, dass** sie 7,5 bis 27,5 Gew.-%, vorzugsweise 10 bis 25 Gew.-%, weiter bevorzugt 12,5 bis 22,5 Gew.-%, noch weiter bevorzugt 15 bis 20 Gew.-% und insbesondere 16 bis 17,5 Gew.-% PPG-14 Butylether enthält.

3. Zusammensetzung nach einem der Ansprüche 1 oder 2, **dadurch gekennzeichnet, dass** sie 1,0 bis 10 Gew.-%, bevorzugt 1,25 bis 7,5 Gew.-%, weiter bevorzugt 1,5 bis 5 Gew.-% und insbesondere 1,75 bis 3,5 Gew.-% Propylenglycoldibenzoat enthält.

4. Zusammensetzung nach einem der Ansprüche 1 bis 3, **dadurch gekennzeichnet, dass** sie als Ölkomponente c3) 1 bis 40 Gew.-%, vorzugsweise 5 bis 39 Gew.-%, weiter bevorzugt 10 bis 38 Gew.-%, noch weiter bevorzugt 15 bis 37 Gew.-%, weiter bevorzugt 20 bis 36 Gew.-% und insbesondere 25 bis 35 Gew.-% Cyclopentasiloxan enthält.

5. Zusammensetzung nach einem der Ansprüche 1 bis 4, **dadurch gekennzeichnet, dass** das Gewichtsverhältnis von c1) zu c2) 1 zu 1 bis 20 zu 1, vorzugsweise 1 zu 1 bis 10 zu 1, weiter bevorzugt 1 zu 1 bis 5 zu 1, noch weiter bevorzugt 1 zu 1 bis 2 zu 1 und insbesondere 1 zu 1 bis 1,5 zu 1 beträgt.

6. Zusammensetzung nach einem der Ansprüche 1 bis 5, **dadurch gekennzeichnet, dass** der Gewichts-Anteil von Ölkomponenten mit einem Brechungsindex ≥1,465 weniger als 45 Gew.-%, vorzugsweise weniger als 40 Gew.-% des Gesamtgewichts aller Ölkomponenten c) ausmacht.

7. Zusammensetzung nach einem der Ansprüche 1 bis 6, **dadurch gekennzeichnet, dass** der schweißhemmende Wirkstoff ausgewählt ist aus mindestens einem Aluminiumzirconiumsalz.

8. Zusammensetzung nach einem der Ansprüche 1 bis 7, **dadurch gekennzeichnet, dass** der mindestens eine Antitranspirant-Wirkstoff in einer Menge von 5 - 40 Gew.-%, bevorzugt 10 - 35 Gew.-%, besonders bevorzugt 15 - 28 Gew.-% und außerordentlich bevorzugt 23-27 Gew.-%, enthalten ist, bezogen auf das Gesamtgewicht der kristallwasserfreien Aktivsubstanz (USP) in der Zusammensetzung.

9. Zusammensetzung nach einem der Ansprüche 1 bis 8, **dadurch gekennzeichnet, dass** sie als Ölkomponente c3) mindestens ein flüchtiges Nichtsiliconöl, das bevorzugt ausgewählt ist aus C₈-C₁₆-Isoparaffinen, insbesondere aus Isodecan, Isoundecan, Isododecan, Isotridecan, Isotetradecan, Isopentadecan, und Isohexadecan, sowie Mischungen hiervon, in einer Gesamtmenge von 10 - 50 Gew.-%, bevorzugt 20 - 40 Gew.-%, besonders bevorzugt 25 - 30 Gew.-%, jeweils bezogen auf die gesamte Zusammensetzung, enthält.

10. Nicht-therapeutisches, kosmetisches Verfahren zur Reduzierung und/oder Regulierung der Schweißbildung und/oder des Körpergeruchs, bei dem eine Zusammensetzung nach einem der Ansprüche 1 bis 9 in einer wirksamen Menge auf die Haut, bevorzugt auf die Haut im Achselbereich, aufgetragen wird.

11. Verwendung von Mischungen aus
c1) PPG-14 Butylether und
c2) mindestens einem Öl aus der Gruppe der Alkylbenzoate, der Alkylendibenzoate, der alkoxylierten Alkylbenzoate, der Polyalkylenoxid-Dibenzoate oder deren Mischungen
mit der Maßgabe, dass der Gewichts-Anteil von Ölkomponenten mit einem Brechungsindex ≥ 1,465 weniger als die Hälfte des Gesamtgewichts der Mischung ausmacht,
und weiterhin mit der Maßgabe, dass die Zusammensetzung - jeweils bezogen auf ihr Gewicht ggf. nach Abzug flüchtiger Treibmittel - 0,75 bis 15 Gew.-% Propylenglycoldibenzoat enthält,
zur Verhinderung oder Verringerung sichtbarer Rückstände von Antitranspirant-Zusammensetzungen.

12. Verwendung gemäß Anspruch 11, **dadurch gekennzeichnet, dass** die Antitranspirant-Zusammensetzungen solche gemäß einem der Ansprüche 1 - 9 sind.

## Claims

1. An antiperspirant composition for personal hygiene, manufactured in the form of a non-aerosol, a stick, a soft solid, a cream, a gel, a non-sprayable or sprayable suspension, a non-sprayable solution, or impregnated on a substrate, containing, based in each case on the weight of said composition, if appropriate after discounting volatile propellants,
a) at least one antiperspirant active substance,
b) from 0 to 7 wt.%, preferably from 0 to 3 wt.%, free water,
c) a mixture of at least two oils, which are liquid under normal conditions, as a carrier,
wherein the composition contains
c1) from 5 to 30 wt.% PPG-14 butyl ether and
c2) from 0.5 to 20 wt.% of at least one oil from the group of alkyl benzoates, alkylene dibenzoates, alkoxylated alkyl benzoates, polyalkylene oxide dibenzoates, or mixtures thereof,
with the proviso that the weight proportion of oil components having a refractive index ≥ 1.465 adds up to less than half of the total weight of all of the oil components c), and furthermore with the proviso that the composition contains from 0.75 to 15 wt.% propylene glycol dibenzoate.

2. The composition according to claim 1, **characterized in that** said composition contains from 7.5 to 27.5 wt.%, preferably from 10 to 25 wt.%, more preferably from 12.5 to 22.5 wt.%, even more preferably from 15 to 20 wt.%, and in particular from 16 to 17.5 wt.% PPG-14 butyl ether.

3. The composition according to one of claims 1 or 2, **characterized in that** said composition contains from 1.0 to 10 wt.%, preferably from 1.25 to 7.5 wt.%, more preferably from 1.5 to 5 wt.%, and in particular from 1.75 to 3.5 wt.% propylene glycol dibenzoate.

4. The composition according to one of claims 1 to 3, **characterized in that** said composition contains, as oil component c3), from 1 to 40 wt.%, preferably from 5 to 39 wt.%, more preferably from 10 to 38 wt.%, even more preferably from 15 to 37 wt.%, more preferably from 20 to 36 wt.%, and in particular from 25 to 35 wt.% cyclopentasiloxane.

5. The composition according to one of claims 1 to 4, **characterized in that** the weight ratio of c1) to c2) is from 1:1 to 20:1, preferably from 1:1 to 10:1, more preferably from 1:1 to 5:1, even more preferably from 1:1 to 2:1, and in particular from 1:1 to 1.5:1.

6. The composition according to one of claims 1 to 5, **characterized in that** the weight proportion of oil components having a refractive index ≥ 1.465 adds up to less than 45 wt.%, preferably less than 40 wt.% of the total weight of all of the oil components c).

7. The composition according to one of claims 1 to 6, **characterized in that** the antiperspirant active ingredient is selected from at least one aluminum zirconium salt.

8. The composition according to one of claims 1 to 7, **characterized in that** the at least one antiperspirant active substance is contained in an amount of from 5 to 40 wt.%, preferably from 10 to 35 wt.%, particularly preferably from 15 to 28 wt.%, and exceptionally preferably from 23 to 27 wt.%, based on the total weight of the active substance (USP), free from water of crystallization, in the composition.

9. The composition according to one of claims 1 to 8, **characterized in that** said composition contains, as oil component c3), at least one volatile non-silicone oil that is preferably selected from C₈-C₁₆ isoparaffins, in particular from isodecane, isoundecane, isododecane, isotridecane, isotetradecane, isopentadecane and isohexadecane, and mixtures thereof, in a total amount of from 10 to 50 wt.%, preferably from 20 to 40 wt.%, particularly preferably from 25 to 30 wt.%, based in each case on the entire composition.

10. A non-therapeutic cosmetic method for reducing and/or regulating sweat formation and/or body odor, in which method a composition according to one of claims 1 to 9 is applied in an effective amount to the skin, preferably to the skin in the axillary area.

11. The use of mixtures of
c1) PPG-14 butyl ether and
c2) at least one oil from the group of alkyl benzoates, alkylene dibenzoates, alkoxylated alkyl benzoates, polyalkylene oxide dibenzoates, or mixtures thereof,
with the proviso that the weight proportion of oil components having a refractive index ≥ 1.465 adds up to less than half of the total weight of the mixture,
and furthermore with the proviso that the composition contains, based in each case on the weight thereof, if appropriate after discounting volatile propellants, from 0,75 to 15 wt.% propylene glycol dibenzoate,
for preventing or reducing visible residues of antiperspirant compositions.

12. The use according to claim 11, **characterized in that** the antiperspirant compositions are those according to one of claims 1-9.

## Revendications

1. Composition antitranspirante pour le soin du corps, confectionnée sous forme de nanoaérosol, de stick, de solide mou, de crème, de gel, de suspension pulvérisable ou non ou imprégnée sur un substrat, contenant - rapporté à son poids, éventuellement après extraction de vecteur volatile :
a) au moins un agent actif antitranspirant,
b) 0 à 7 % en poids, de préférence 0 à 3 % en poids d'eau libre
c) un mélange d'au moins deux huiles liquides en conditions normales, comme vecteur, la composition contenant :
c1) 5 à 30 % en poids de PPG 14 butyléther, et
c2) 0,5 à 20 % en poids d'au moins une huile issue du groupe des benzoates d'alkyle, des benzoates d'alkyle alcoxylés, des dibenzoates de polyoxyde d'alkylène ou de leurs mélanges,
à condition que la fraction pondérale des composants huileux d'indice de réfraction
≥ 1,465 soit inférieure à la moitié du poids total des composants huileux c),
et à condition, en outre, que la composition contienne 0,75 à 15 % en poids de dibenzoate de propylène glycol.

2. Composition selon la revendication 1, **caractérisée en ce qu'**elle contient 7,5 à 27,5 % en poids, de préférence 10 à 25 % en poids, plus préférentiellement 12,5 à 22,5 % en poids, encore plus préférentiellement 15 à 20 % en poids et en particulier 16 à 17,5 % en poids de PPG 14 butyléther.

3. Composition selon la revendication 1 ou 2, **caractérisée en ce qu'**elle contient 1,0 à 10 % en poids, de préférence 1,25 à 7,5 % en poids, plus préférentiellement 1,5 à 5 % en poids et en particulier 1,75 à 3,5 % en poids de dibenzoate de propylène glycol.

4. Composition selon une des revendications 1 à 3, **caractérisée en ce qu'**elle contient comme composants huileux c3) 1 à 40 % en poids, de préférence 5 à 39 % en poids, plus préférentiellement 10 à 38 % en poids, encore plus préférentiellement 15 à 37 % en poids, plus particulièrement 20 à 36 % en poids et en particulier 25 à 35 % en poids de cyclopentasiloxane.

5. Composition selon une des revendications 1 à 4, **caractérisée en ce que** le rapport pondéral de c1) à c2) est de 1:1 à 20:1, de préférence 1:1 à 10:1, plus préférentiellement 1:1 à 5:1, encore plus préférentiellement 1:1 à 2:1, et en particulier 1:1 à 1,5:1.

6. Composition selon une des revendications 1 5, **caractérisée en ce que** la proportion en poids de composants huileux d'indice de réfraction supérieur à 1,465 est inférieur à 45 % en poids, de préférence inférieur à 40 % du poids total de tous les composants huileux c).

7. Composition selon une des revendications 1 à 6, **caractérisée en ce que** l'agent actif antitranspirant est choisi par au moins un sel d'aluminium et de zirconium.

8. Composition selon une des revendications 1 à 7, **caractérisés en ce que** l'au moins un agent antitranspirant est contenu à hauteur de 5 à 40 % en poids, de préférence 10 à 35 % en poids, particulièrement préférentiellement 15 à 28% en poids et tout particulièrement préférentiellement 23 à 27 % en poids, rapporté au poids total de la substance active sans eau de cristallisation (USP) dans la composition

9. Composition selon une des revendications 1 à 8, **caractérisée en ce qu'**elle contient comme composants huileux c3) au moins une huile non de silicone, choisie de préférence parmi les C₈₋₁₆-isoparaffines, en parmi l'isodécane, l'isoundécane, l'isododécane, l'isotridécane, l'isotetradécane, l'isopentadécane et l'isohexadécane, ainsi que leurs mélanges, à hauteur de 10 à 50 % en poids, de préférence de 20 à 40 % en poids, particulièrement préférentiellement 25 à 30 % en poids, rapporté préférentiellement à la composition totale.

10. Procédé cosmétique non-thérapeutique de réduction et/ou de régulation de la transpiration et/ou de l'odeur corporelle, dans lequel la composition selon une des revendications 1 à 9 est appliquée dans une quantité efficace sur la peau, de préférence sur la peau des aisselles.

11. Utilisation de mélanges de :
c1) PPG 14 butyléther, et
c2) au moins une huile du groupe des benzoates d'alkyle, des benzoates d'alkyle alcoxylés, des dibenzoates de polyoxyde d'alkylène ou de leurs mélanges,
à condition que la fraction pondérale des composants huileux d'indice de réfraction
≥ 1,465 soit inférieure à la moitié du poids total du mélange,
et à condition, en outre, que la composition contienne 0,75 à 15 % en poids de dibenzoate de propylène glycol, rapporté à son poids éventuellement après extraction de vecteur volatile,
pour empêcher ou réduire les résidus visibles de compositions antitranspirantes.

12. Utilisation selon la revendication 11, **caractérisée en ce que** les compositions antitranspirantes sont celles selon une des revendications 1 à 9.
